(19) Europäisches Patentamt
European Patent Office
Office européen des brevets

(11) **EP 2 792 354 A2**

(12) **EUROPEAN PATENT APPLICATION**
published in accordance with Art. 153(4) EPC

(43) Date of publication:
**22.10.2014 Bulletin 2014/43**

(51) Int Cl.:
*A61K 31/205* (2006.01)     *A61K 31/221* (2006.01)
*A61K 31/664* (2006.01)     *A61K 31/198* (2006.01)
*A61K 31/045* (2006.01)     *A61P 25/00* (2006.01)
*A61P 1/16* (2006.01)

(21) Application number: **12774566.9**

(22) Date of filing: **04.05.2012**

(86) International application number:
**PCT/RU2012/000355**

(87) International publication number:
**WO 2012/144938 (26.10.2012 Gazette 2012/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.03.2011 RU 2011109884**

(71) Applicant: **Obshchestvo S Ogranichennoj
Otvetstvennostju
«Biotekhnologii Pyshchino»
Moskovskaya obl. 142290 (RU)**

(72) Inventor: **DINNIK, Vladimir Vladimirovich
Pushchino
Moscovskaya obl. 142290 (RU)**

(74) Representative: **Wablat Lange Karthaus
Anwaltssozietät
Potsdamer Chaussee 48
14129 Berlin (DE)**

(54) **COMPOSITION OF PROTECTORS OF ACUTE AND CHRONIC HEPATIC ENCEPHALOPATHY AND METHOD FOR TREATING ACUTE AND CHRONIC HEPATIC ENCEPHALOPATHY**

(57)     The invention relates to medicine and concerns pharmaceutical compositions (formulations) which can be made also, including in the form of dietary supplements, having the properties of protectors of hepatic encephalopathy and hepatoprotectors , including :
- Acute (fulminant) hepatic encephalopathy caused by acute liver failure ;
- Chronic hepatic encephalopathy, in which the underlying etiology - cirrhosis of the liver caused by alcoholic , non-alcoholic or viral hepatitis , etc.

*The invention consists in that:* the treatment of acute and chronic hepatic encephalopathy include complex compositions, whose components have a synergistic effect on various metabolic and signaling systems , including the activation of: osmoprotectant and energetic systems; detoxification reactions of ammonium ion and other toxins ; antioxidant systems of brain and liver cells, and of other tissues and organs.

Compositions for the treatment of acute hepatic encephalopathy contain as a base : L- carnitine , acetyl -L-carnitine and phosphocreatine , and may also contain :

N- acetylcysteine and ethanol (1); succinate , L-glutamate , L- arginine, N- acetylcysteine and betaine (2); succinate , L- glutamate , L- arginine, and betaine (3);

Compositions for the treatment of chronic hepatic encephalopathies, as bases comprise : L-carnitine , acetyl -L- carnitine , succinate , L- glutamate , L- arginine, betaine, and phosphocreatine , and may also contain : N- acetylcysteine, coenzyme Q10 and dihydroquercetin (4); S- adenosyl methionine , coenzyme Q10 and dihydroquercetin (5); S- adenosyl methionine , coenzyme Q10, dihydroquercetin and lipoamide (6).

The invention provides the means (preparations) to expand the arsenal of the treatment of the treatment of acute and chronic hepatic encephalopathy,the means hindering the development of coma and death in acute hepatic encephalopathy and providing reverse development of behavioral, neurological and mental disorders in chronic encephalopathy.

**Description**

*1.INTRODUCTION*

The invention relates to medicine and can also be used in the food industry in the form of biologically active dietary supplements , and in the pharmaceutical industry to meet the challenges of treatment of hepatic encephalopathy (HE) : providing reverse development of behavioral, neurological and mental disorders, to prevent the development of coma and lethal outcome in patients. For the treatment of acute and chronic hepatic encephalopathy are presented complex composite formulations, whose components have a synergistic effect on various metabolic and signaling systems , including activation of: osmoprotectant and energetic metabolic systems; detoxification reactions of ammonium ion and of other toxins ; antioxidant systems of the brain and liver cells , and of other tissues and organs.

HE - reversible metabolic and signaling dysfunction of the brain (resulting in the spectrum of neuropsychiatric disorders) caused by the failure of the liver. As the basis of acute HE is severe dysfunction of the liver - the liver failure to provide detoxification of ammonium ion ($NH_4^+$) and other toxins entering the brain, bypassing the liver. Mandatory feature of acute HE is hyperammonemia (1.2).

Chronic HE often occur in liver cirrhosis caused by alcoholic or non-alcoholic steatohepatitis , viral hepatitis and other factors. Severe chronic forms of HE (stages III and IV) and acute HE can often lead to coma and death of the organism (1-6).

According to modern definitions (1-5) may be distinguished three types of HE related to:

**A -** acute (acute) liver failure ;
**B -** the presence of porto- systemic shunt (by pass), without liver disease ;
**C -** cirrhosis (cirrhosis).

In accordance with the criteria of West Haven , classify minimal and four (1-4) stages of HE : ME - Minimal (latent) HE, no visible signs of HE in the patients with cirrhosis;

1 (I) - with an inversion of sleep, asterix , and others;
2 (II) - with lethargy (apathy), disorientation in time and space, inconsistent speech , etc.;
3 (III) - sleepiness (stupor), complete disorientation , eccentric behavior , hyperreflexia, etc.;
4 (IV) - coma.

Preclinical stages of HE- ME , and the stages I and II ,are diagnosed using special psychometric and neuropsychological tests, and stage III and IV - clinically (2). 60-80 % of patients with cirrhosis have HE, half of them have the stages I-IV of HE, and the annual risk of HE in patients with cirrhosis is 20 % (3.4).

Coma occurring in acute HE, is usually associated with: the development of vasogenic or toxic edema ; the dysfunction of brain ; swelling of astrocytes and (or) with endothelial dysfunction and impaired blood-brain barrier permeability (4,7,8).

In the previous decade, the prevailing view (7,9-12,130) was ,that excessive activity of the ionotropic glutamate receptors (NMDA), arising in the presence of excess $NH_4^+$, causes : the accumulation of Ca2 + and activation of neuronal NO synthase (nNOS), with subsequent accumulation of NO, cGMP and activation PKG; activation of Na-K ATPase and ATP depletion ; reduction of the concentration of glutamate and glutamine accumulation ; astrocyte swelling and , thus , the development of toxic edema (swelling).

L-glutamine is considered as the main toxic osmolyte (13,14), which is formed by glutamine synthase in the astrocytes:

$$\text{L- Glutamate + NH3 + ATP} \rightarrow \text{L- glutamine + ADP + Pi.} \qquad \text{(A)}$$

Output of astrocytes basic organic osmolytes from astrocytes - of myo - inositol, taurine and betaine in these conditions, does not compensate for the accumulation of glutamine ("Trojan horse" hypothesis). This may lead: to an increase in cell volume ,due to the entrance of water ; to the oxidative and nitrosative stress ; to the formation of permeability transition mitochondrial pores ,due to hydrolysis of glutamine and $NH_4^+$ accumulation in the mitochondria (14).

Recently, the most popular is the idea, that the swelling of astrocytes and brain edema are associated with: the development of oxidative and nitrosative stress; the activation of NADPH- oxidase (15) and NF$\kappa\beta$ (16) ; activation of arachidonic acid metabolism (17) and depletion of reduced glutathione - GSH (18,19). It is believed that nitrosative stress leads to reduced activity of glutamine synthase in the brain (A), one of the key enzymes of ammonia ($NH_4^+$) clearance in the body (20,21) . It is also known ,that the presence of inflammatory processes result in the activation of inducible iNOS (2,4,7,8,12,23,116).

The oxidation of NAD- dependent substrates in the mitochondria of brain cells is suppressed in HE (12.28). It was also found a significant decrease in ATP (12,29,30,124) and the accumulation of lactate and glutamine in the brain (12,29,30,48). The old hypothesis ,that $NH_4^+$ may substitute for K + in a number of transport processes (24,25) and thus may cause depolarization of brain cells, was experimentally confirmed only recently (26,27). Metabolic and signaling pathways that are involved in the mechanism of toxic action of $NH_4^+$ insufficiently studied yet.

In acute liver failure , for example, when there is portal hypertension and shunting of hepatic blood flow , the excess of $NH_4^+$ and other toxins produced by the micro flora of the gastrointestinal tract (at gastrointestinal bleeding),-may lead to dysfunction of the brain and the development of acute HE. These secondary toxins may include: fatty acids , mercaptans , manganese, false neurotransmitters tyramine and oktapamina type (1.33), peripheral benzodiazepine receptor ligands (BDZ) - and other neurosteroids, etc. (2-4,7,8,33).

In the case of severe chronic HE (stages III and IV), swelling and dysfunction of astrocytes are not always present, or vice versa, a hypertrophy and hyper function of astrocytes may take place. However, in any case, oxidative and nitrosative stress is considered as a main cause of brain dysfunction in HE (1-4,7,8,15).

This may result in the deregulation of different neural networks in the brain, associated: with hyper activation (acute HE) or inhibition (chronic HE) of the signaling chain- glutamate => NMDA => Ca2 + => cGMP => PKG (9-11); in violation of the system of exchange of monoamines (34) ;with disturbances in signaling pathways involving acetylcholine (Ach) (35,36); with the dominance of signaling pathways, involving inhibitory transmitter gamma-amino butyric acid (GABA), due to excessive activation of peripheral BDZ receptors (4,7,33) and excessive production of neurosteroids in mitochondria (7), etc.

In acute liver diseases ,induced by drug intoxication , primary toxins become activated derivatives thereof (in particular in the form of Coenzyme A derivatives), which : cause rapid steatosis; reduce the level of GSH; produce oxidative stress , hepatic cell necrosis and inhibition of energy production by various cell types (as a result of inhibition of the Krebs cycle , β- oxidation of long chained fatty acids (LCFA) and of the urea cycle (in the liver)). The accumulation of LCFA under these conditions may lead to the development micro vesicular steatosis in liver (37,38). Accumulating CoA derivatives of LCFA in these conditions themselves become toxins, causing further collapse of various energy and transport systems and the death (39.130). This scenario may occur at acetylsalicylic acid poisoning (Reye syndrome and Reyes like diseases (37-39)). Blocking the reaction of the urea cycle in the liver at these conditions ,leads to the development of acute secondary hyperammonemia.

Similar mechanisms may be implemented in a number of congenital diseases, associated with the disturbances in the oxidation of branched chain amino acids and the accumulation of toxic CoA derivatives of branched keto acids (propionic, methylmalonic and isovaleric acidemia , etc.) ( 40,41,130). It should be noted that short chained fatty acids, with an odd number of carbon atoms ( propionic and valeric acids) formed by intestinal bacteria , can also be important secondary toxins reinforcing the toxic effects of excess $NH_4^+$ (2,33). The role of such secondary toxins not taken into account.

The list of underappreciated secondary toxins may also include LCFA (myristic , palmitic, oleic , linoleic and arachidonic acids), which may accumulate , at the excess of $NH_4^+$, due to the hydrolysis of triglycerides or of membrane phospholipids , as holds for heart attack or stroke (ischemia / reperfusion), when a local increase in the concentrations of such toxic LCFA can reach tens of micro molar (42,43). It was shown (44, 45), that palmitoylcamitine at concentrations of 10-20 microM, may cause the death of cells of different types, due to the disturbances in $Ca^{++}$ homeostasis (activation of reticular $Ca^{++}$ channels resulting in $Ca^{++}$ accumulation and subsequent $Ca^{++}$ dependent phospholipases activation) followed by the activation of lipoxygenase and arachidonic acid oxidation pathways and cell death ,due to formation of nonspecific cationic permeability of the plasma membrane. LCFA amplify the toxic effects of $NH_4^+$ (46).

This is important, because the significant part of patients with the history of HE has alcoholic or non-alcoholic steatohepatitis.

Under such circumstances, it becomes clear that a lack of strict correlation between NH4 + level in the blood and the severity of HE symptoms can be linked with heterogeneous population of patients and with the presence of secondary toxins in the brain and liver (fatty acid , neurosteroids and others) . In one study conducted in India , with a homogeneous cohort of patients with acute HE , it was shown strong correlation between NH4 + level in the blood, the severity of HE and the risk of death of patients (47).

Thus, it is now recognized that ammonium, inflammation and infection underlie the pathogenesis of HE in liver cirrhosis , and their synergistic action may take place (4,7,8).

Existing approaches to the treatment of patients with HE are well known (1-6). Effective and the last way to treat acute and severe HE is liver transplantation (1-4).

In acute HE, caused by poisoning of the liver with acetaminophen (often used by suicide), the most effective means is the administration of N-acetyl cysteine (NAC), which helps to relieve oxidative stress , to restore the pool of GSH and to suppress the production of certain inflammatory cytokines (49).

*In the practice of the treatment of acute and chronic HE associated with an excess of ammonium ion, within the last 60 years are used two combined approaches (1-6,33,50 -55):*

1. reduction of production of $NH_4^+$ by intestinal bacteria 6using lactulose and probiotics;

2. activation of the systems of ammonia clearance in the body , primarily due to the activation of the urea cycle in the liver and of glutamine synthase in various tissues and organs.

The treatment of HE may also include the application of antagonists of GABA receptors , transfusion therapy or radical method - liver transplantation (1-4).

There are two proprietary drugs having primarily to reduce circulating NH4 + in the body ,which are declared as hepatoprotectors, means of dealing with hyperammonemia and hepatic encephalopathy protectors :

1. The preparation "HepaMerz"(Germany) (52).
It is used more than 15 years in clinical world practice. As parts of "HepaMerz»: L- ornithine and L- aspartate, thus its name in research circles is LOLA. Daily intake at acute HE : 20 - 40g, sometimes - to 80g .
2. " Glutargin " (53) - Ukrainian drug developed in 2001. "Glutargin" from 2005 is used as a drug . As part of" "Glutargin»: L- glutamate and L- arginine. Recommended daily intake of up to 6 - 10g

The effect of both drugs are similar and primarily aimed at the activation of the main system cleaning NH4 + in the liver - urea cycle and glutamine synthase.
Both compositions , LOLA and "Glutargin" contain inter convertible compounds.
L- arginine and L- ornithine are substrates (intermediates) of the urea cycle. L- aspartate , ATP and carbamoyl phosphate are cosubstrates of the cycle. Total balance equation in the urea cycle is as follows:

$$NH4 + + HCO_3 -+ 3ATP_{-4} + H2O + L\text{- aspartate} \rightarrow urea + 2AMP_{-3} + AMP + + PPi + 2Pi + H + + fumarate \qquad (B)$$

Glutamate-oxaloacetate transaminase reaction is fast quasi-equilibrium , which quickly transforms L-glutamate in L- aspartate :

$$L\text{- glutamate} + oxaloacetate = L\text{- aspartate} + \alpha\text{- ketoglutarate} \qquad (C)$$

L- glutamate is a source of L- ornithine in another transaminase reactions:

$$+ L\text{- glutamate} -\gamma\text{- glutamate semi aldehyde} = L\text{- Ornithine} + \alpha\text{- ketoglutarate} \qquad (D)$$

L- glutamate being used in glutamine synthase reaction in the brain, muscle, kidney and in perivenous hepatocytes ,is involved in lowering of NH4 + with the formation of glutamine (reaction A).
L- arginine is also the substrate ofNO-synthases (NOS) and the source of NO in the organism:

$$L\text{- arginine} + 2NADPH + 2O_2 \rightarrow L\text{- citrulline} + 2NADP + +2H_2O + NO \qquad (E)$$

The combined use of probiotics , L- glutamate and (or) L-arginine in the treatment of PE, was investigated in the 50- 60s (55). In the 60s in a number of patents and research works to accelerate cleaning NH4, instead of the pair L- glutamate and L- arginine , it was suggested to use the L-ornithine and L- aspartate (51).

Composition" Glutargin " was not widely used in the world of medical practice, although actively marketed in Ukraine as a hepatoprotector , antioxidant and anti-alcohol drug (53).

Composition "HepaMerz" (LOLA) has been actively studied in various medical centers, including the Russian Federation (1-6,56-62), but data on its efficacy are very contradictory. Sometimes there is a marked reduction of NH4 + combined with the improvement of mental state of patients ( 5,56-58), sometimes- reversion only of mild forms of HE (5.59). Other studies have not confirmed the effectiveness of LOLA (60-62). In Russia registered a patent for a method of treating patients with HE using lactulose , probiotics , and LOLA (63).

Recently, attempts are being made to use the combination of L- ornithine and phenyl acetate and others (64). Several clinical studies have used L- carnitine or acetyl -L- carnitine (2 doses 4g.), which also possess protective properties with milder forms of HE (minimal HE and the stages I, II). Sometimes their use is followed by marked decline in NH4 + in the blood and by the improvement of mental state of patients (65-67).

Separate group are drugs with claimed hepatoprotective properties. These drugs aimed at restoring the liver functions of patients with cirrhosis and hepatitis of different etiology.

Presented recently in the domestic market "Phosphogliv" includes phospholipid phosphatidylcholine and glycyrrhizic acid (flavolignan of thistle) (6,68). In world practice of medicine and physiology and in sport have long used similar composition (dietary supplement «Liver-Pro» ,company «Santegra», USA). It is believed ,that some flavonoids and phospholipids substantially strengthen the antioxidant defense mechanisms and reparative processes in liver cells. Recently were patented means, which in addition to the above compounds include bile acids, and other compounds (69). Currently in the treatment of such diseases considered new approach aimed at metabolic activation Stellate and Kupffer cells of the liver, involving cannabinoid receptors modulation.

Thus, in the world of medical practice, the most popular composition addressed for HE treatment is LOLA ,that is taken by us as a prototype.

The main drawback of the existing approaches in the search for methods of correction of HE, are attempts to find a minimum set of tools ,that can effectively protect the body from excess of NH4, both in acute and chronic HE.

This applies to attempts to use: LOLA (1-6,51-63), "Glutargin"(53), L- carnitine (65-67,70,71 ),the inhibitors of NMDA (10) or GABA receptors (33,50), combinations of NMDA receptor inhibitors and L- carnitine (128), inhibitors of NOS (129), inhibitors of glutamine synthase or glutaminase (13,14), the introduction of an encapsulated glutaminase in the blood (125,126), creatine (127), etc.

*There is no one - two compounds can be cure in terms of HE (55).*

By the definition of HE are metabolic HE. HE - are reversible complex metabolic abnormalities in the brain caused by primary disorders of the liver (1-4).

Acute and chronic PE may be considered as "metabolic" syndrome, which includes complex chemical (metabolic) and mental (signaling) disorders , correction of which requires complex protection.

Leaving aside the existing effective ways to reduce the production of NH4 + by bacteria of the gastrointestinal tract, in our opinion , effective protectors of metabolic and signaling disorders in acute and chronic HE should be complex and have to have the following properties :

    1. activate osmoprotectant systems of brain and liver cells , involved in the regulation of cell volume and facilitate the removal of "toxic" osmolytes: glutamine, alanine and lactate , i.e. impeding the development of cerebral edema.

    2. activate energy metabolism of mitochondria of different cells to support active operation of: glutamine synthase (A), urea cycle (B), transport ATP ases (involved in the regulation of Ca++ and Na + / K + / Cl- homeostasis in the brain , kidney, liver and muscle) - all energy wasting processes.

    3. activate the removal of NH4 + in the brain, kidney, muscle and liver (perivenous hepatocytes) with glutamine synthase, as well as in the liver with participation of the urea cycle (periportal hepatocytes) ;

    4. reduce the activities of different neural networks signaling, involving excitatory neurotransmitters glutamate (NMDA, AMPA) and activate inhibitory transmitters signaling with GABA and glycine (in cases of acute HE) . Restore the activity of arginine => NO => cGMP => PKG signaling pathway and of signaling pathways involving acetylcholine (in terms of chronic HE).

    5. activate the antioxidant and anti-inflammatory system of brain and liver cells.

    6. reduce the accumulation of toxic CoA derivatives and other toxic substances.

    7. induce metabolic and signaling systems , aimed at the transcriptional activation and de novo synthesis of enzymes of a number of metabolic pathways , including: oxidative phosphorylation of mitochondria , the Krebs cycle , the urea cycle , glutamate exchange, GSH synthesis, pentose shunt, etc. (for chronic HE) .

Long-term search of protectors of HE ,using thousands of animals ,allowed us to find an unexpected results consists in the fact that a combination of some metabolites have synergistic effects of their actions on various metabolic and signaling systems. The combination of relatively low concentrations of these compounds increases the effect of each action.

*This applies to the combined action of :*

-   Succinate , glutamate and acetyl-L- carnitine- on energy metabolism of cells;
-   Biguanidines (L- arginine and creatine) and trimethylamines (L- carnitine, acetyl -L- carnitine and betaine) -on various signaling and transport system with the involvement of central α2 and m2 receptors;
-   Succinate , Coenzyme Q10 ,lipoamide ,L- arginine and L- glutamate -on activation of various transcriptional factors of metabolic and signaling pathways.
-   L- glutamate , N- acetyl cysteine (or S- adenosyl methionine) and betaine -on activation of antioxidant cellular systems ;
-   L- glutamate and acetyl -L- carnitine -on activation of N- acetylglutamate synthesis (key activator urea cycle) and on activation of N acetylaspartylglutamate synthesis (NAAG) - inhibitory dipeptide- ligand of metabotropic glutamate receptors mGluRII (2), etc.

*Characteristic components of the proposed formulations protectors PE.*

All component parts are natural compounds have been well studied and are standard components of food and pharmaceutical products.

**L- caruitiue , acetyl -L- carnitine and betaine.**

*L- carnitine* - coenzyme participating in the transfer of acyl groups in the cells of plants and animals with a carbon chain length of C2 (acetate) and C3 (propionate) to C24 -26 . L- carnitine is an essential buffer of acyl groups. Because of this, exogenous L- carnitine is able to reduce the concentration of toxic AcylCoA - derivatives of drugs and bile acids and increase the concentration of free Coenzyme A.

L-carnitine properties are discussed in an enormous number of publications. It is indicated for use:in cardiovascular diseases , obesity and type 2 diabetes , in HE , pain syndromes , in the treatment of neurological diseases in children, in patients with myocardial stroke. Widely used in sports and is recommended as burner of the weight.

Mechanisms and signaling pathways involved in the regulation of the volume of cells are poorly understood. L- carnitine was one of the first chemicals, the effect of which increased animal survival in in animal models of acute hyperammonemia (70), although this was not always confirmed (71). It was shown that all quaternary amines , including L- carnitine, acetyl-L- carnitine and betaine are osmoprotectants (71.72) and holinomimetics (71-75).

However by itself L- carnitine, even at high concentrations (15 - 30g) is not effective protector of HE.

In our opinion, three important key properties of L- carnitine include:

- Holinomimetic (m2- central cholinergic receptors) and osmoprotectant (together with the effect of $\alpha$2- adrenoceptor agonists - L- arginine and creatine);
- Buffer of acyl groups of substrates ,oxidized in the Krebs cycle in forms of acetyl - or sussinylCoA, i.e. member of energy metabolism ;
- Acceptor of acyl groups , etc. ,of toxic derivatives of drugs ,of LCFA and other compounds metabolized in the form of CoA derivatives (including bile acids).

In the present compositions of the present invention, the amount of L-carnitine per 1 dose is 4 - 6g or 2.5 - 3g (acute HE) or 1.5 - 2g (chronic HE).

*Acetyl -L- carnitine,*

As well as L- carnitine, it is widely used in medicine and sports. It is known that in the context of acute HE (HE models) there is a strong decrease in the rate of oxidation of NAD-dependent substrates in the Krebs cycle , including pyruvate , malate, palmitoylcarnitine and others in the mitochondria , in comparison with the oxidation of succinate and glutamate (12,28).

In the state of reduced oxidation rates of pyruvate and ketone bodies and of LCFA (in HE), the formation of acetyl-L- carnitine (of acetylCoA) - is of great importance for high-speed operation of the Krebs cycle and generation of ATP in the mitochondria (in the presence of glutamate and succinate) .

Use in the claimed compositions of acetyl-L- carnitine has several objectives :

- The action as holinomimetic (m2- central cholinergic receptors) and osmoprotectant (together with the action of $\alpha$2- receptors agonists - L- arginine and creatine);
- Donor of acetyl groups in the synthesis of Ach in the neuronal presynaptic terminals;
- Donor of acetyl groups in the Krebs cycle (acetyl formation and activation of the Krebs cycle ; in combination with glutamate and succinate);
- Donor of acetyl groups in the synthesis of inhibitory peptide transmitter NAAG (with glutamate) ;
- Acetyl group donor in the synthesis of N- acetyl glutamate formation (with glutamate), - of a key activator of the urea cycle.

In the claimed compositions of the present invention, the amount acetyl -L- carnitine used at a doses: of 4 - r 5 g, or 1 - 2g (acute HE) and 0.5 - 1.5 g (chronic HE).

*Betaine (trimethylglycine)* is widely used: in the food industry in an amount of 0.3 - 3g (as a weight gainer); as hepatoprotector ; in trauma ;in treatment of bowel diseases and other. Betaine is a decay product of choline, a precursor of glycine and serine. Bipolar molecule capable of retaining water. Along with myo- inositol and taurine , it is an endogenous osmolyte because under conditions of accumulating toxic osmolytes glutamine , lactate and alanine - exit of betaine and other osmolytes prevents an enormous increase in cell volume.

Betaine - a good donor of methyl groups , which is of great importance in various methylation reactions , including the induction of synthesis of enzymes of various metabolic pathways de novo. Along with L- carnitine and acetyl-L- carnitine, betaine is holinomimetic (75,78).

Betaine thus introduced into the compositions as:

- Osmoprotectants and cholinomimetic;
- Donor of methyl groups.

In the present compositions of the present invention, the amount of betaine (trimethyl glycine) for 1 dose of 0.5 - 2g (acute and chronic HE).

## L- arginine, L- glutamate

*L-Arginine* - one of the intermediates of the urea cycle , the substrate in the reactions of NO and polyamines synthesis. Precursor of creatine. Arginine is" multi faced Janus ", which is written not less than about carnitine. Is widely used in medicine and sports (like gainer) .

Known "arginine paradox" (89), which is caused by the fact that an increase in the concentration of arginine more 1mM results in NOS activation and rise of NO, in spite of high affinity (of n, e and i) NOS to arginine (15mkM). Such a property is explained by the presence of a competitive inhibitor of NOS - asymmetric dimethylarginine (ADMA) in the cells (89). The concentration of ADMA in the conditions of various types of abnormalities may reach 100 - 300mkM (90,91). However, other mechanisms of action of arginine may be involved too. It is known that arginine has some affinity for $\alpha$2- adrenoreceptors, since the introduction of arginine in the blood causes the hypotensive effect (92). It is also shown (31,32) that L- arginine , through $\alpha$2 adrenergic receptors causes the activation of eNOS in myocardiocytes , increasing signaling : G$\beta\gamma$ => PIP3K => ACT => eNOS. L- arginine may act in synergy with norepinephrine (NE) on adipocytes (78).

Thus, administration of L- arginine in the claimed compositions provides:

- Activation (substrate of urea cycle and activator N- acetyl glutamate synthase), i.e. of the urea cycle flux in the liver ;
- Activation of central $\alpha$2 adrenergic receptors, which (together with the m2 acetylcholine receptors ) may be important:
- In osmoregulation of astrocytes and neurons;
- In presynaptic inhibition in different neural networks;
- In the suppression of signaling, involving cAMP and Ca2 + .
- Activation and restoration of signaling in the chain

NO => cGMP => PKG (in conditions of chronic HE).

- Restoration of creatine pool.
- The activation of PIP3K, AKT, mTOR signaling network, controlling the synthesis of various proteins (when reparative processes and angiogenesis).

As stated the compositions of the present invention, the amount of L-arginine per 1 dose of 1.5 - 2.5 g (for acute and chronic HE) .

*L- glutamate* - a key amino acid that participates in transamination's with different amino acids (i.e., source of aspartate and ornithine in the urea cycle), excitatory neurotransmitter in the brain , the precursor of the inhibitory transmitters GABA and peptide NAAG. It is well oxidized in the Krebs cycle, being paired with succinate (in state 3 by B.Chance)- holds maximal respiration rate). The source for $\alpha$- ketoglutarate in the Krebs cycle (via glutamatedehydrogenase and transaminase reactions (B , C)) and the substrate for the synthesis of glutamine in glutamine synthase reaction ( A). Under conditions of acute and chronic HE the concentration of glutamate decreases in combination with glutamine accumulation and development of edema (4,7,12,93-95).

Thus , the presence of L- glutamate in the claimed compositions provide:

- Activation of glutamine synthase (reaction A) ;
- Activation of the urea cycle, due to the formation of an important regulator of the Cycle - N- acetyl glutamate (with acetyl-L- carnitine);
- Activation of the urea cycle through the formation of co-substrate of the cycle L- aspartate (reaction B) ;
- Activation of the Krebs cycle by reutilization of oxaloacetate (reaction B) -which is Krebs cycle substrate and an inhibitor of succinate dehydrogenase , thus glutamate helps to achieve the maximum rate of oxidation of succinate in the Krebs cycle (with succinate and acetyl -L- carnitine);

- Activation of the Krebs cycle by preventing possible depletion of $\alpha$- ketoglutarate (reaction B, C)
- Synthesis of inhibitory transmitter GABA and inhibitory dipeptide NAAG (in conjunction with acetyl -L- carnitine) involved in presynaptic inhibition by metabotropic glutamate receptor type II - mGluR II (2) (87,88);
- Activation of signaling involving mTOR and protein synthesis ;
- Participation in the synthesis of GSH (together with N- acetyl cysteine and betaine).

In the present compositions (for acute and chronic PE) of the present invention, the amount of L-glutamate (glutamic acid) at 1 dose is 0.75 - 1.5 g.

*Succinic acid (succinate)* - one of the Krebs cycle intermediates ,the substrate of succinate dehydrogenase reaction (SDH). SDH is one of the fastest reactions of the Krebs cycle and , in contrast to key regulatory reactions of Krebs Cycle ($\alpha$-ketoglutarate dehydrogenase and citrate synthase reactions)- SDH rate depends weakly on NAD / NADH and ATP / ADP ratios in the mitochondria. Inhibition of SDH by various AcylCoA's is also weak. Therefore , in conditions of hypoxia and other pathological conditions , succinate oxidation is particularly important. Paired with glutamate succinate support maximal rate of mitochondrial respiration (12) . Under conditions of acute HE, mitochondrial respiratory depression for this pair of substrates may be expressed much weaker than for all NAD- dependent substrates (pyruvate, malate, ketone bodies , palmitoylcarnitine, etc.) (12,28).

The role of succinic acid at various conditions, was widely discussed in many papers , on symposia and conferences (96.97).

Succinic acid is widely used in medicine and sports. Even low pharmacological amount of succinic acid or its derivatives (e.g. Meksidol or ammonium succinate) have an important regulatory effects on the metabolism of humans and animals (97) . This may be due to its action through GPR91 cell receptors, coupled with Gi / Go and Gq proteins (98). Activation of GPR91 expression by succinate occurs during ischemia. The important role of succinic acid has also stabilizing hypoxia inducible factor HIF- 1 , ensuring efficient energy production by mitochondria in conditions of hypoxia and under different stress conditions , including oxidative stress (99,100).

Thus, succinic acid provides :

- High rate of the Krebs cycle operation and of mitochondrial ATP production(in combination with glutamate and acetyl -L- carnitine);
- Efficient energy production under conditions of oxidative and nitrosative stress;
- Induction of metabolic pathways de novo (via HIF and receptors GPR91), providing efficient supply of $FADH_2$ and NADH.

However, by itself ,succinic acid, even at high concentrations (up to 5-10 mg / kg), is not an effective protector of acute ammonia intoxication (101).

In the present compositions of the present invention, the amount of succinate (succinic acid) for 1 dose is 0.75 - 1.5 g.

*Phosphocreatine* (CrP)- an important cofactor for the transfer of high-energy phosphate groups of ATP and cell ATP buffer :

$$CrP + ADP = ATP + creatine$$

(F)

CrP is very important at maximal anaerobic efforts supplying ATP ,as total creatine pool in muscle and brain cells is 4 - 5 times higher than the total content of adenylates. Widely used in sports. In terms of HE has a decay of creatine to creatinine and serum creatinine increased (4.93).

As part of claimed compositions , CrP is used for:

- Recovery of creatine + phosphocreatine pool in the cells;
- As biguanidine , which through $\alpha2$ adrenergic receptors may participate in cellular osmoregulation and in neuronal presynaptic inhibition (with L- arginine and cholinomimetics) .

In large doses , phosphocreatine may also work as osmoprotectant , in combination with L-carnitine and ethanol (to replace L- arginine ,see below).

In the present compositions of the present invention, the amount of phosphocreatine at 1 dose is 4 - 6g (acute HE) or 0.5 - 1.5 g (chronic HE)

*Ethanol* is one of the most popular compounds which, when is chronically consumed may lead to addiction and fatty liver (alcoholic steatohepatitis), due to the reduction of NAD and sustained suppression ofNADH oxidation (of NAD- dependent substrates). In acute administration of large doses of ethanol acetaldehyde poisoning may occurs. However , in some cases with acute HE , produced by acetaminophen , the administration of ethanol shows positive effect in combination with N- acetyl cysteine (102).

In acute HE, with hyperammonemia, ethanol can be used as a regulator of NMDA, GABA and glycine receptors , because even small doses of ethanol activate inhibitory GABAa receptors , and large doses (50mM and above) lead to the inhibition of the NMDA- receptors (80.123), and glycine receptors activation (123).

In the present compositions of the present invention, the amount of ethanol per 1 dose is 20 - 50 ml .

*N-acetyl cysteine (NAC) -* acetylated amino acid cysteine. Plays an important role in :glutathione synthesis reactions (GSH metabolic pathway, de novo), being substrate, like glutamate and glycine ; in various reactions of trans sulfuration.

Under conditions of acute HE and oxidative and nitrosamine stress (2,4,8,12,15), the activities of antioxidant systems of the liver and brain cells fall. The decrease the activities of glutathione peroxidase and glutathione reductase is marked (12). In experimental models of HE with the introduction of acetaminophen or other toxins , it was shown that there is a drop of reduced GSH, due to the development of oxidative stress (103,104). In the experiments performed on cultures of neurons and astrocytes it also was shown ,that cell death due to oxidative stress is associated with the depletion of GSH (18,19).

GSH levels may be reduced , both due to its transformation into GSSG in antioxidant reactions , and by the formation of conjugates with various toxins or in the reactions of leukotriene synthesis ,due to activation of arachidonic acid metabolic and signaling pathways.

NAC is used as an antidote for poisoning by acetaminophen, providing active restoration of glutathione level and recovery of GSH (103,104). Combinations of NAC, glutamate and glycine-are also able to provide an efficient resynthesis of GSH de novo. Using tripeptide GSH is impractical because first it breaks in the kidneys, and then starting compounds used by the body for the resynthesis of GSH (105).

Therefore, as part of protectors of HE, NAC:

- Provides , in combination with L- glutamate the resynthesis of GSH;
- Participates in the reactions of trans sulfuration;
- Regulates anti-inflammatory processes.

However, by itself NAC, even at high concentrations (15 - 20 g) is not an effective protector of acute PE caused by primary hyperammonemia.

As stated the compositions of the present invention, the amount of N-acetyl cysteine at a dose of 1 - 3g. (acute HE) or 0.5 - 1.5 g. (chronic HE).

### CoenzymeQ10 and dihydroquercetin (DHQ)

Presented antioxidants may reduce oxidative stress, even in terms of delayed recovery and low pool of GSH and low pentose phosphate shunt activity in cells.

*CoenzymeQ10* and its derivatives are widely used:in medical practice, in the various food supplements, in aging programs , as well as cardio protectants and nootropic agents, as anticancer agents, as protectors of mitochondrial myopathies and neurodegenerative diseases, etc. CoenzymeQ10 is involved in the regulation of hundreds of genes , in the regulation of mitochondriogenesis and of anti-inflammatory processes , etc.(106,107).

As part of protectors of HE ,Q10 may support the induction of mitochondriogenesis (in combination with succinate) and suppression of action of inflammatory factors (TNF$\alpha$, etc.)

In the present compositions of the present invention, the amount CoenzymeQ10 per 1 dose is 20 - 30mg (chronic HE).

*DHQ* - flavonoid extracted from larch - well-known and widely used antioxidant (108) - is used as a part of the protectors of their immediate purpose - as an antioxidant. DHQ may be replaced BY flavonoids, obtained from grapes (Resveratrol (109)) or from green tea.

In the present compositions of the present invention, the amount of DHQ for 1 dose of 15 - 20mg (chronic HE).

### S- adenosyl -L- methionine (SAM)

*SAM* - one of the most important cofactors of cellular metabolism , participating in dozen reactions: of trans methylation (phospholipids, proteins, nucleic acids and neurotransmitters); of trans sulfuration, with its own cycle of reactions , with the formation of homocysteine , combined with the synthesis of GSH; of the synthesis of polyamines.

SAM is used : as a dietary supplement; for treatment of osteoporosis , traumas and liver diseases ; as well as an antidepressant; a stabilizer of cell membranes and as the regulator of cell growth and repair processes. In recent years, SAM is also applied as an antidote to acetaminophen or CC14 poisoning (49), as well as a regulator of reparative processes in the liver (49.110).

As part of the protectors of chronic PE, introduction SAM provides:

- Restoration of the pool of GSH (together with glutamate) and induction of reactions of GSH synthesis de novo;
- Participation in the stabilization of cell membranes , nucleic acids, proteins , etc.

In the present compositions of the present invention the amount of SAM per 1 dose is 0.1 - 0.5 g. (chronic HE).

### $\alpha$- *Lipoic acid and Lipoamide*

$\alpha$- *Lipoic (thioctic acid)* is widely used in food industry as a food additive and conserving, as well as in medicine, as an antioxidant and anti-inflammatory compound. $\alpha$- Lipoic acid is involved in dozens of reactions of oxidation of $\alpha$-keto acids ,in the regulation of activity of PPAR nuclear factors and of signaling involving cAMP (111).

*Lipoamide* - amide form $\alpha$- lipoic acid, is more effective regulator of transcription and translation of nuclear factors PPAR$\alpha$, PPAR$\gamma$ and CPT1$\alpha$ It is the inductor of synthesis of eNOS and an activator of signaling pathway : eNOS => cGMP => PKG. Lipoamide suppresses the accumulation of reactive oxygen species , prevents the depletion of NAD and others (112,113).

As part of protectors PE, lipoamide provides:

- Activation of mitochondriogenesis and of NAD- dependent substrates oxidation (with Q10 and succinate);
- Regulation of the expression of PPAR;
- Regulation of the signaling pathway eNOS => cGMP => PKG (together with L- arginine).

In the present compositions of the present invention, the amount of Lipoamide per 1 dose is 0.03 - 0.1 g. (chronic HE).

### 2. Compositions and administration of the claimed compositions of protectors of HE

The following examples serve to illustrate the claimed invention and not to limit the scope of the claims. All component parts are well studied , are natural compounds and are widely used in food and pharmaceutical products.

Suggested formulations protectors of HE can be used also as a dietary supplements and executed in the form of powders or granules, suitable for oral administration. Claimed compositions of HE may be prepared by their mixing directly , divided into portions and granulated or divided into portions and packaged in sachets ,using conventional techniques and the auxiliary components according to the standard for this technology.

Formulations may be used as a beverage with the addition of the required quantities of sodium bicarbonate (equimolar to the concentrations of acids .) 1 dose of the proposed formulations are dissolved in 150 - 250ml water to make an acceptable drink.

Compositions of protectors of HE may be orally administered at 1 - 2 doses up to 2 - 3 times a day, depending on the extent of the patient's condition, weight and the stage of HE .

Coenzyme Q10 and dihydroquercetin (DHQ) may be dissolved in a vegetable oil (preferably olive oil), and packaged into soft gelatin capsules of standard for this field of technology and used in combination with the basic composition of the treatment of chronic HE in the same way. Dihydroquercetin and Q10, packed in $\beta$- cyclodextrin or in other water-soluble forms ,can be administered in the main composition in the appropriate amounts , without dissolving in the oil.

### Examples of claimed formulations of protectors of HE :

### *Compositions of protectors of acute HE.*

*Example 1.* Sachet (granules or powder), weighing 18gramms(g).

```
L-Carnitine                5g
Acetyl -L- carnitine       5g
phosphocreatine            6g
N- acetyl -L- cysteine     2g
additionally
40ml ethanol.
```

Composition (sachet) dissolved in 250 - 300ml of water , adding ethanol (in the absence of contraindications i.e. , acute alcoholic hepatitis, etc.).

Ingredients are used in the case of acute poisoning , the reception per os from 1 to 3 doses.

```
Example 2.                           EXAMPLE 3 . ;
L- carnitine          3g.    L- carnitine            1.5 g
Succinate 1g          1g.    Acetyl -L- carnitine    1.5 g
L- glutamate          1g.    succinate               0.8 g
L- Arginine           1.5 g  L- glutamate            1 g
Phospho creatine      2g.    L- Arginine             1.25 g
                             N- acetyl -L- cysteine  1g
                             Betaine                 1g.
```

Sachet (granules or powder) .

Composition (sachet) dissolved in 150 - 200 ml of water. Use per os of 3 to 6 doses in acute HE.

### Compositions of protectors of chronic HE.

Are in the form of powders or granules in sachets.

```
Example 4.                               Example 5 .
L- carnitine              2g.    L- carnitine              1.5g
Acetyl -L- carnitine      1g.    Acetyl -L- carnitine      1.5 g
Succinate                 1g     succinate                 1g
L- glutamate,             1g.    L- glutamate              1.25 g
L- arginine,              2g.    L- arginine               1.5 g
Betaine                   0.5g.  Betaine                   1g
S- adenosyl -L- methionine 0.5g  S- adenosyl -L- methionine 0.25 g
                                 phosphocreatine           1g.
                                 Lipoamide                 0.05 g


Optional                         Optional


In the form of soft capsules.    In the form of soft capsules.
CoenzymeQ10               20mg    CoenzymeQ10               30mg
DHQ                      15mg     DHQ                      20mg
```

Q10 and DHQ may be in the form of water-soluble forms ($\beta$-cyclodextrin , etc.) and added to the main composition of protectors. Composition sachet dissolved in 150 - 200 ml of water. Receiving per os 1 - 2 doses of 2 - 3 times a day.

### 3.TREATMENT OF HE

### Animal Models of HE

In the experiments, male albino mice weighing 20 - 35 g after 12 hours of fasting.

*1. Classical model of hyperammonemia* (9-12,71), with the introduction i.p. of lethal doses of $NH_4Cl$, with simultaneous injection of NaCl. The defined lethal dose of $NH_4Cl$ (LD100) was 16.5 mmol/kg. After the occurrence of clonic and tonic convulsions ,followed by coma , the animals died within 10 - 15 minutes.

A solution of $NH_4Cl$ (pH 7.4, NaOH) and solutions of hepatoprotectors were prepared on the basis of administration of 25 - 30ml/kg solutions i.p. Taking into account the steep dependence of the percent survival of animals on injected dose of NH4Cl (at values close to LD100), in acute experiments $NH_4Cl$ doses administered, were taken at 110 - 115 % of the LD100, and were 18.0 - 18.5 mmol / kg (LD100 +). This allows to avoid the influence of non-specific factors on the survival of animals, when the protectors of HE are applied. The protectors tested in most experiments with acute HE were administered i.p. 20 - 30 seconds after $NH_4Cl$ injections. Sequences of administration of protectors and toxins are listed in the tables.

*2. Animal models with liver failure*

Because the liver of healthy animal does not reflect the state of animal with liver failure , like in various types of HE, a few well-known models (114,115) of acute or chronic liver failure were used , resulting in the development of HE . The recommended classification of the stages of HE for laboratory animals was used (116).

*2.1 The models of acute liver failure* , resulting in necrosis and fatty degeneration of the liver cells due to oxidative stress , by introducing :$CCl_4$ (2 - 4ml/kg subcutaneously or i.p.); acetaminophen-AAF (300 - 500mg/kg i.p.) or thioacetamide (TAA , 300 - 500mg / kg i.p.), in accordance with known data (103,104,116-118). The model of acute multi organ failure , with i.p. introduction of propionic acid (119,120).

*2.2 Chronic liver failure:* caused by introduction of $CCl_4$ (or TAA), followed by 24 hours $NH_4Cl$ injection, or use a model for type 2 diabetes (115) followed by injection of $CCl_4$ (or TAA).

2.2.1 *The models of obesity* in animals which , along with the standard feed , fed lard (controlled lipid composition) at the rate of 300 - 500 mg for 1 pet per day. Control the level of glucose, insulin , ALT , bilirubin, glycerol, triglycerides and fatty acids in the blood and in the liver, histological examination of the liver was carried out.

2.2.2 *The model of type 2 diabetes,* according to the data of (115), in which after 1 - 2 months of feeding fat, animals were injected i.p. 50mg/kg of streptozotocin (to suppress the activity of $\beta$-cells) and then 15 days later , after controlling for the main indicators of the blood ,were used in the experiments.

*Examples of formulations of protectors of HE in animal experiments in vivo:*

Composition protectors acute HE in mg per 1 kg of animal weight:

$\Sigma$1 (*Example 1*): L- carnitine = 2000 ,Acetyl -L- carnitine = 750 -2000 ,phosphocreatine -2000, N-acetyl -L- cysteine = 1000, 3 ml ethanol.

$\Sigma$2 (*Example 2*): L- carnitine = 1000 , succinate=300 , L- glutamate = 375 , L- arginine = 500 , phosphcreatine=450. *LOLA1*: L- ornithine = 1280 , L- aspartate = 960.

Composition protectors chronic PE mg per 1 kg of animal weight:

$\Sigma$3 (*Example 4*): L- carnitine = 350 , acetyl -L- carnitine = 150 , succinate=150 , L- glutamate = 200 , L- arginine=250 , betaine = 250 , S- adenosyl -L- methionine = 100; Q10 = 20 and DHA =15(in oil). *LOLA2:* L- ornithine = 750 , L- aspartate = 500 .

**Treatment of acute HE**

Summary of the data of acute HE treatment with indicated protectors are shown in Table 1. It is evident that the simultaneous administration of toxin $NH_4Cl$ and different protectors may result in survival of part or of all animals. Composition, having no urea cycle intermediates ($\Sigma$1), the effect of which is aimed at osmoprotectant mechanisms and activation of central $\alpha$2 and m2 receptors - ensures the survival of part of animals (in the range 40, 70 %). The effect varies and does not depend on sex of the animals. Introduction of LOLA, the effect of which is aimed at reducing the concentration of $NH_4Cl$ (in the body), ensures the survival of 60-80 % of the animals. Best performing composition was $\Sigma$2 (70,100%). Introduction of half doses of both protectors ,15 minutes before the administration of $NH_4Cl$, provides higher efficiency of the compositions $\Sigma$2 and LOLA (Tab.1 6.7). However , the model of acute intoxication with ammonium of animals with an intact liver, does not correspond to the state of the defence mechanisms in animals with liver failure.

Preliminary introduction of propionic acid suppresses the energy metabolism of the cells of different organs and tissues , as well as of $NH_4+$ detoxification reactions ,due to the accumulation of toxic derivatives (of propionylCoA and metilmalonylCoA) (119.121) and is a model of propionic and methylmalonic acidemia and of multiple organ failure. When administered to animals at doses 20-25mmol/kg -propionate Na leads to drop of ATP in the cells and in an

accumulation of $NH_4^+$ in the blood, due to the inhibition of the urea cycle (92). Table 1 (row 8) shows that the introduction of non-lethal doses of $NH_4Cl$ in such conditions lead to rapid death of all animals. Even with large doses of $NH_4Cl$, introduction of protectors tested ,ensures the survival of the animals (Table 2, lines 10,11). The best protection produces the composition $\Sigma2$ which leads to survival of 60,80% of treated animals.

Table 1. (lines 12-14) also shows summary data obtained on HE models in which non-lethal doses of hepatotoxic $CCl_4$, cause steatohepatitis and liver cell necrosis ,that simulates the presence of liver failure. Introduction of various doses of NH4 (12-14 mmol / kg) at 24 hours after administration of these toxins, can cause the development of acute HE and death of most or all animals. However, this effect depends on the dose used toxins $CCl_4$. It can be seen that low doses of $CCl_4$ (2ml/kg), being administered to intact animals , results in almost total protection against the lethal dose of $NH_4$, resulting in the survival of 80 % of treated animals, i.e. there is the effect of preconditioning.

It is also known that at the introduction of $CCl_4$, AAF or TAA , repair processes begin in a few hours. Therefore, the choice of the models and conditions to demonstrate the effectiveness of the protectors should be carefully monitored , since at low doses of a variety of toxins , protectors may have a positive impact ,as it can be clearly seen from a number of published experiments ( 56,117,118).

In our conditions, the introduction of protectors in varying degrees ,ensures the survival of part or of most animals. The most effective composition is $\Sigma2$.

### Treatment of chronic HE

With a large dose of CC14 (or TAA), the subsequent introduction of non-lethal doses of $NH_4Cl$ after 24 hours (Table 2, line 1) led to the death of all animals within 3-4 days , with the development of signs of chronic HE for the animals (loss of activity , lethargy , lack of response to painful stimuli, and coma).

Introduction of protectors $\Sigma3$ and LOLA, 24 hours after $CCl_4$ (1 per day), resulted in a decrease in the death of part of animals and prevented the development of symptoms of chronic HE. The composition $\Sigma3$ had better efficacy, resulting in the survival of 70% of treated animals (Table 2 line 2.3).

And finally, in a model of type 2 diabetes , non-lethal dose of $CCl_4$ =2ml/kg (for healthy animals), lead to death of diabetic animals within 3-4 days , due to the development of hyperammonemia and coma , with all known signs of chronic HE. Daily administration of protectors also causes the survival of some part of the animals. Composition $\Sigma3$ again proves to be more effective than LOLA (Tab. 2, lines 5,6).

Table 3. shows the parameters of the blood of the animals with type 2 diabetes , resulting in the control and diabetic animals (prior to $CCl_4$ administration) and in diabetic (D2) animals monitored 72 hours after the administration of $CCl_4$ or subsequent administration of protectors . The comparison shows that the composition $\Sigma3$ has a better efficiency.

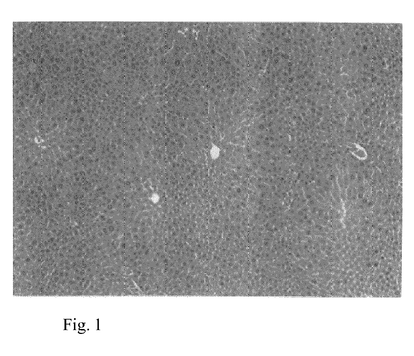

Chronic toxicity of compositions $\Sigma2$ and $\Sigma3$ was determined in experiments with the introduction of these protectors for 1 month to intact animals per os. Blood parameters were normal, histological changes in the liver (as an example, $\Sigma3$, Fig. 1) and brain were absent. These data show that the tested compounds are not toxic.

### 4. Identifier the following set of features that achieve results.

*The claimed compositions of the followihg options are:*

1. Composition of protectors of acute and chronic hepatic encephalopathy, containing per single dose :L- carnitine =4-6 g. ; acetyl -L- carnitine = 4- 5g. ; phosphocreatine = 4-6 g. ; N- acetyl cysteine= 1- 3g. ; ethanol - 20 -50ml.

2. Composition of protectors of acute and chronic hepatic encephalopathy, containing per single dose: L- carnitine = 1.5-3 g. ; acetyl -L- carnitine =1-3 g.; Succinate -=0.75-1.5 g. ; L- glutamate = 0.75-1.5 g. ; L- arginine = 1,5-2,5 g.; phosphocreatine =2-3g .; N- acetyl cysteine = 0.25-1.5 g.; betaine = 0.25 -2g .; creatinine phosphate =0,25- 1 .

3. Composition of protectors of acute and chronic hepatic encephalopathy containing per single dose :L- carnitine=2.5- 3 g. ; acetyl -L- carnitine =0.5-1 g.; Succinate = 1-1.5 g ; L- glutamate = 1-1.5 g. ; L- arginine = 1.5 -2 g.; phosphocreatine=1-3 g.

4. Composition of protectors of acute and chronic hepatic encephalopathy, containing per single dose :L- carnitine = 1.5-3 g.; acetyl -L- carnitine =1.3 g. ;Succinate = 0.75-1.5 g ; L- glutamate = 0.75-1.5 g ; L- arginine = 1,5-2,5 g ;phosphocreatine = 2-3 g. ; N- acetyl cysteine = 0.25-1.5 g. ; betaine = 0.25- 2g. ; creatinine phosphate= 0,25- 1 g ; Coenzyme Q10 =20 - 30mg and dihydroquercetin = 15 - 20mg .

5. Composition protectors acute and chronic hepatic encephalopathy containing per single dose: L-carnitine = 1.5-3 g.; acetyl -L- carnitine = 1.3 g.; Succinate = 0.75-1.5 g.; , L- glutamate = 0.75-1.5 g.; L- arginine = 1,5-2,5 g.; betaine = 0.25 -2g .; phosphocreatine =2-3 g .; creatinine phosphate= 0 ,25- 1 g ; Coenzyme Q10 = 20 - 30mg ; dihydroquercetin = 15 - 20mg and S- adenosylmethionin = 0,1-0,5 g, while the composition may further contain 0.03-0.1 g lipoamide

For the treatment of acute and chronic HE, and various liver diseases selected from acute and chronic hepatitis and cirrhosis and steatohepatitis of liver of different etiologies : Orally administered to patients for treatment of acute stages of the above formulations of the compositions 1 , 2 and 3 in an amount of from 1 to 3 doses in any combination

Depending on the patient's weight and tolerance of the individual components of the composition. When treating chronic stages of the above formulations of the compositions 4 and 5 in an amount of from 1 to 6 doses per day , in any combination depending on the weight of the patient and individual tolerance of components of the composition.

| Table 1. Effect of protectors on animal survival in acute HE, induced by ammonia injection in mice in vivo. | | | | | | |
|---|---|---|---|---|---|---|
| No | Pretreatment: injected* compounds and time to injection of $NH_4Cl$ | Toxin $NH_4Cl$ and doses: | Protectors** and doses | Mice injected | Time to death (min) | % survival |
| 1 | no | LD100 (16,5 mmol/kg wt) | NaCl | 10 | 10-15 | 0 |
| 2 | -||- | LD100+ (18,5 mmol/kg wt) | NaCl | 10 | 8-12 | 0 |
| 3 | -||- | LD100+ | $\Sigma_1$ | 10, 10 | 20-40 | 40, 70 |
| 4 | -||- | LD100+ | $\Sigma_2$ | 10, 10 | 15-20 | 70, 100 |
| 5 | -||- | LD100+ | LOLA | 10, 10 | 15-20 | 60, 80 |
| 6 | $\Sigma_2$ (1,25 g/kg; 15 min) | LD100+ | NaCl | 10 | 12-20 | 100 |
| 7 | LOLA (1,25 g/kg; 15 min) | LD100+ | NaCl | 10 | 12-20 | 70 |
| 8 | Propionate Na (25 mmol/kg; 15 min) | LD30 (14 mmol/kg) | NaCl | 10, 10 | 15-20 | 0 |
| 9 | -||- | LD100 | NaCl | 10 | 8-12 | 0 |
| 10 | -||- | LD100 | $\Sigma_2$ | 10, 10 | 20-40 | 60, 80 |
| 11 | -||- | LD100 | LOLA | 10, 10 | 20-30 | 30, 40 |
| 12 | $CCl_4$ (2 ml/kg wt; 24 hours) | LD100+ | NaCl | 10 | 10-12 | 80 |
| 13 | $CCl_4$ (3 ml/kg wt; 24 hours) | LD100+ | NaCl | 10 | 15-30 | 50 |
| 14 | $CCl_4$ (4 ml/kg wt.; 24 hours) | LD100+ | NaCl | 10 | 7-12 | 0 |
| * Intraperitoneal (i.p.) injection; **Injected i.p. within 30sec after toxin. | | | | | | |

| The table of the comparison 2. Effect of protectors of chronic HE in the experiments on mice in vivo. | | | | | | |
|---|---|---|---|---|---|---|
| No | Animals condition and pretreatment | | Toxin* and doses: | Protector** and doses: | Mice injecte | Time to death (hours) after toxin injection | % survival, 150 hours |
| 1 | Control mice | $CCl_4$ (4 ml/ kg) | $NH_4Cl$ (10 mmol/ kg wt.) | NaCl | 10 | 72-96 | 0 |
| 2 | -||- | -||- | -||- | $\Sigma_3$ | 10, 10 | 72-120 | 40,50 |
| 3 | Control mice + $CCl_4$ | -||- | -||- | LOLA wt.) | 10, 10 | 72-120 | 30,40 |
| 4 | Diabetic mice | no $CCl_4$ | $CCl_4$ (2 ml/ kg) | NaCl | 20 | 72-96 | 0 |
| 5 | Diabetic mice | -||- | -||- | $\Sigma_2$ | 20 | 84-120 | 60 |
| 6 | Diabetic mice | -||- | -||- | LOLA | 20 | 84-108 | 20 |

(continued)

| The table of the comparison 2. Effect of protectors of chronic HE in the experiments on mice in vivo. | | | | | | |
|---|---|---|---|---|---|---|
| No | Animals condition and pretreatment | | Toxin* and doses: | Protector** and doses: | Mice injecte | Time to death (hours) after toxin injection | % survival, 150 hours |
| *Injected 24 hours after CCl$_4$; **injected 24hours after NH$_4$Cl or CCl$_4$, once a day for 3 days. | | | | | | |

The table of the comparison 3.

The comparison of the efficacy of compositions $\sum_3$ and LoLa on the alive diabetic animals treated with CCL4 and protectors used (according to pp.4-6 of Tab.2)

| | AAT plasma U/L | Glucose Plasma, mMol | LCFA Plasma, mMol |
|---|---|---|---|
| Control | 65$\pm$20 | 6,4$\pm$0,5 | 0,53$\pm$0,12 |
| Type 2 diabetes (D2) | 96$\pm$26 | 16,6$\pm$1,5 | 1,95$\pm$0,38 |
| D2, CCl$_4$, injection of NaCl 2 days. | 4280$\pm$510 | 18,3$\pm$1,6 | 2,05$\pm$0,44 |
| D2, CCl$_4$, injection of NaCl 3 days. | 1540$\pm$350 | 13,1$\pm$0,8 | 1,55$\pm$0,28 |
| D2, CCl$_4$, injections of LOLA, 3 days. | 2860$\pm$440 | 15,2$\pm$1,4 | 1,75$\pm$0,43 |

The table of the comparison 3.

The comparison of the efficacy of compositions $\sum_3$ and LoLa on the alive diabetic animals treated with CCL4 and protectors used (according to pp.4-6 of Tab.2)

| | AAT plasma U/L | Glucose Plasma, mMol | LCFA Plasma, mMol |
|---|---|---|---|
| Control | 65$\pm$20 | 6,4$\pm$0,5 | 0,53$\pm$0,12 |
| Type 2 diabetes (D2) | 96$\pm$26 | 16,6$\pm$1,5 | 1,95$\pm$0,38 |
| D2, CCl$_4$, injection of NaCl 2 days. | 4280$\pm$510 | 18,3$\pm$1,6 | 2,05$\pm$0,44 |
| D2, CCl$_4$, injection of NaCl 3 days. D2, CCl$_4$, injections of LOLA, 3 days. | 1540$\pm$350 2860$\pm$440 | 13,1$\pm$0,8 15,2$\pm$1,4 | 1,55$\pm$0,28 1,75$\pm$0,43 |

**Bibliography:**

1. Bajaj S. Review article: the modern management of hepatic encephalopathy. Aliment Pharmacology Ther 2010; 31(5): 537-547 pp.

2. Wolf DC. Encephalopathy, hepatic. eMedicine Gastroenterology, Aug 18, 2010. http://emedicine.medscape.com/article/186101-overview

3. A1 Sibae M.R., McGuire B.M. Current trends in the treatment of hepatic encephalopathy. Ther Clin Risk Manag. 2009 Jun;5(3):617-26 pp.

4. Seyan A.S., Hughes R.D., Shawcross D.L. Changing face of hepatic encephalopathy: role of inflammation and oxidative stress. World J Gastroenterol. 2010 July 21; 16(27): 3347-3357 pp.

5. Ivashkin V.T., Nadinskaya M.Y., AO Buyeverov A.O. Hepatic encephalopathy and its metabolic correction methods. Bolez. org. indigestion (rus). 2001, 3: 25-7 c.

6. Strekalova O.S., Uchaikin V.F. ,Ipatova O.M., Torhovskaya T.P., N.V .Medvedev, Storozhakov G.I., Archakov, A.I. Comatose states: etiopathogenesis, experimental study, treatment of hepatic coma. Biomedical Chemistry (rus). 2009, 55 (4): 380-396 c.

7. Butterworth R.F. Phatogenesis of hepatic encephalopathy: new insights from neuroimaging and molecular studies. J Hepatol 2003; 39: 278-285 pp.

8. Bjerring P.N., Eefsen M., Hansen B.A., Larsen F.S. The brain in acute liver failure. A tortuous path from hyperammonemia to cerebral edema. Metab Brain Dis. 2009 Mar;24(1):5-14 pp.

9. Marcaida G., Miñana M.D., Burgal M., Grisolia S., Felipo V. Ammonia prevents activation of NMDA receptors by glutamate in rat cerebellar neuronal cultures. Eur J Neurosci. 1995 Dec 1;7(12):2389-96 pp.

10. Rodrigo R, Cauli O, Boix J, ElMlili N, Agusti A, Felipo V. Role of NMDA receptors in acute liver failure and ammonia toxicity: therapeutical implications. Neurochem Int. 2009 Jul-Aug;55(1-3):113-8pp.

11. Montoliu C., Llansola M., Kosenko E., Corbalán R., Felipo V.Role of cyclic GMP in glutamate neurotoxicity in primary cultures of cerebellar neurons. Neuropharmacology. 1999 Dec;38(12):1883-91 pp.

12. Kosenko E.A., Kaminsky Y.G .,Cellular mechanisms of toxicity of ammonia. Moscow Publ LCI (rus), 2008, 288 c.

13. Brusilow S.W., Traystman R. Hepatic encephalopathy. N Engl J Med. 1986 Mar 20;314(12):786-7 pp.

14. Albrecht J., Norenberg M.D. Glutamine: a Trojan horse in ammonia neurotoxicity. Hepatology. 2006 Oct;44(4):788-94 pp.

15. Reinehr R., Görg B., Becker S., Qvartskhava N., Bidmon H.J., Selbach O., Haas H.L., Schliess F., Häussinger D. Hypoosmotic swelling and ammonia increase oxidative stress by NADPH oxidase in cultured astrocytes and vital brain slices. Glia. 2007 May;55(7):758-71 pp.

16. Sinke A.P., Jayakumar A.R., Panickar K.S., Moriyama M., Reddy P.V., Norenberg M.D. NFkappaB in the mechanism of ammonia-induced astrocyte swelling in culture. J Neurochem. 2008 Sep;106(6):2302-11 pp.

17. Jayakumar A.R., Rama Rao K.V., Tong X.Y., Norenberg M.D. Calcium in the mechanism of ammonia-induced astrocyte swelling. J Neurochem. 2009 May; 109 Suppl 1:252-7 pp.

18. Hilgier W., Wegrzynowicz M., Ruszkiewicz J., Oja S.S., Saransaari P., Albrecht J. Direct exposure to ammonia and hyperammonemia increase the extracellular accumulation and degradation of astroglia-derived glutathione in the rat prefrontal cortex. Toxicol Sci. 2010 Sep;117(1):163-8 pp.

19. Gegg M.E., Beltran B., Salas-Pino S., Bolanos J.P., Clark J.B., Moncada.S., Heales S.J. Differential effect of nitric oxide on glutathione metabolism and mitochondrial function in astrocytes and neurones: implications for neuroprotection/neurodegeneration? J Neurochem. 2003 Jul;86(1):228-37 pp.

20. Suárez I., Bodega G., Fernández B. Glutamine synthetase in brain: effect of ammonia. Neurochem Int. 2002 Aug-Sep;41(2-3):123-42 pp.

21. Kosenko E., Llansola M., Montoliu C., Monfort P., Rodrigo R., Hernandez-Viadel M., Erceg S., Sánchez-Perez A.M., Felipo V. Glutamine synthetase activity and glutamine content in brain: modulation by NMDA receptors and nitric oxide. Neurochemistry international 2003;43(4-5):493-9 pp.

22. Singh S., Trigun S.K. Activation of neuronal nitric oxide synthase in cerebellum of chronic hepatic encephalopathy rats is associated with up-regulation of NADPH-producing pathway. Cerebellum. 2010 Sep;9(3):384-97 pp.

23. Häussinger D., Görg B. Interaction of oxidative stress, astrocyte swelling and cerebral ammonia toxicity. Curr Opin Clin Nutr Metab Care. 2010 Jan;13(1):87-92 pp.

24. Moser H. Electrophysiological evidence for ammonium as a substitute for potassium in activating the sodium pump in a crayfish sensory neuron. Can J Physiol Pharmacol. 1987 Feb;65(2): 141-5 pp.

25. Keicher E., Meech R. Endogenous Na(+)-K+ (or NH4+)-2Cl- cotransport in Rana oocytes; anomalous effect of external NH4+ on pHi. J Physiol. 1994 Feb 15;475(1):45-57 pp.

26. Kelly T., Kafitz K.W., Roderigo C., Rose C.R. Ammonium-evoked alterations in intracellular sodium and pH reduce glial glutamate transport activity. Glia. 2009 Jul;57(9):921-34 pp.

27. Kelly T., Rose C.R. Ammonium influx pathways into astrocytes and neurones of hippocampal slices. J Neurochem. 2010 Dec;115(5):1123-36 pp.

28. Díaz-Muñoz M., Tapia R. Functional changes of brain mitochondria during experimental hepatic encephalopathy. Biochem Pharmacol. 1989 Nov 1;38(21):3835-41 pp.

29. Hawkins R.A., Miller A.L., Nielsen R.C., Veech R.L.The acute action of ammonia on rat brain metabolism in vivo. Biochem J. 1973 Aug;134(4):1001-8 pp.

30. Kosenko E., Kaminsky Y., Grau E., Miñana M.D., Marcaida G., Grisolia S., Felipo V. Brain ATP depletion induced by acute ammonia intoxication in rats is mediated by activation of the NMDA receptor and Na+,K(+)-ATPase. J Neurochem. 1994; 63(6): 2172-8 pp.

31. Nenov M.N., Berezhnov A.V., Fedotova E.I., Grushin K.S., O.J. Pimenov, Semushina S.G., Pakhomov LA., Murashev A.N .,DynnikV.V., Zinchenko V.P., Kokoz Y.M. Activation of α2-adrenoceptors by L-arginine in cardiomyocytes SD and SHR rats. Collection of articles,Conf. "Reception and intracellular signaling." Pushchino, ONTI Press, PSC RAS (Eds. Zinchenko V.P. et al), 2009, 143-148 p.

32. Nenov M.N. Regulation of L-type Ca2 + currents by L-arginine via α2-adrenoceptor activation in isolated ventricular cardiomyocytes.Ph. Diss.. biol. sciences. Pushchino, ITEB RAS.: 2009, 24 p.

33. Basile A.S., Jones E.A., Skolnick Ph. The pathogenesis and treatment of hepatic encephalopathy: evidence for the involvement of benzodiazepine receptor ligands. Pharmacol Rev 1991; 43(1): 27-71 pp.

34. Radad K., Gille G., Rausch W.D. Short review on dopamine agonists: insight into clinical and research studies relevant to Parkinson's disease. Pharmacol Rep. 2005 Nov-Dec;57(6):701-12 pp.

35. Ratnakumari L., Qureshi LA., Maysinger D., Butterworth R.F. Developmental deficiency of the cholinergic system in congenitally hyperammonemic spf mice: effect of acetyl-L-carnitine. J Pharmacol Exp Ther. 1995 Jul;274(1):437-43 pp.

36. García-Ayllón M.S., Cauli O., Silveyra M.X., Rodrigo R., Candela A., Compañ A., Jover R., Pérez-Mateo M., Martinez S., Felipo V., Sáez-Valero J. Brain cholinergic impairment in liver failure. Brain. 2008 Nov;131 (Pt 11):2946-56 pp.

37. Trost L.C., Lemasters J.J. Reye's syndrome and related chemical toxicity. Mitochondria in Pathogenesis 2002; 5: 425-450 pp.

38. Ward M.R. Reye's syndrome: an update. Nurse Pract. 1997 Dec;22(12):45-6, 49-50, 52-3 pp. ,2005, 238-243 c.

39. Dynnik V.V., Jafarov R..D, Grishin E.V., V.A. Kassymov ,Grushin K.S., Kokoz Y.M., Zinchenko V.P. Hepatic encephalopathy. Reyes syndrome. Toxic effects of fatty acids - "four in one". Collection of articles, Conf. "Reception and intracellular signaling", Pushchino, ONTI Press, PSC RAS (Eds. Zinchenko V.P. et al),2005,238-242pp.

40. Knerr I., Weinhold N., Vockley J., Gibson K.M. Advances and challenges in the treatment of branched-chain amino/keto acid metabolic defects. J Inherit Metab Dis. 2011 Feb 3. [Epub ahead of print]

41. Dionisi-Vici C., Deodato F., Röschinger W., Rhead W., Wilcken B. 'Classical' organic acidurias, propionic aciduria, methylmalonic aciduria and isovaleric aciduria: long-term outcome and effects of expanded newborn screening using tandem mass spectrometry. J Inherit Metab Dis. 2006 Apr-Jun;29(2-3):383-9 pp.

42. Dhillon H.S., Dose J.M., Scheff S.W., Prasad M.R. Time course of changes in lactate and free fatty acids after experimental brain injury and relationship to morphologic damage. Exp Neurol. 1997 Jul;146(1):240-9 pp.

43. Takeuchi Y., Morii H., Tamura M., Hayaishi O., Watanabe Y. A possible mechanism of mitochondrial dysfunction during cerebral ischemia: inhibition of mitochondrial respiration activity by arachidonic acid. Arch Biochem Biophys. 1991 Aug 15;289(1):33-8 pp.

44. Berezhnov A.V., Fedotova E.I., Nenov M.N., V.P. Zinchenko , V.V. Dynnik Toxic effects of fatty acids. Role of phospholipases . Collection of articles, Conf. " Reception and intracellular signaling " Pushchino, ONTI Press, PSC RAS (Eds. Zinchenko V.P. et al), 2009 , 15-19 pp.

45. Berezhnov A.V .,Fedotova E.I., Nenov M.N., V.P. Zinchenko , V.V. Dynnik Calcium overload and cardiomyocytes death in the presence of activated fatty acids. Contribution of phospholipases. Biological membranes (rus), Moscow, 2010 - 27 (1), 67-76 pp.

46. Berezhnov AV Investigation of the mechanisms of acute toxic effects of acylcarnithines : Ph. Diss. biol. Sciences ,IBK RAS, Pushchino, 2009.

47. Bhatia V., Singh R., Acharya S.K. Predictive value of arterial ammonia for complications and outcome in acute liver failure. Gut. 2006 Jan; 55(1): 98-104 pp.

48. Kaminsky Y., Kosenko E. Brain purine metabolism and xanthine dehydrogenase/oxidase conversion in hyperammonemia are under control of NMDA receptors and nitric oxide. Brain Res. 2009 Oct 19;1294:193-201 pp.

49. Terneus M.V., Brown J.M., Carpenter A.B., Valentovic M.A. Comparison of S-adenosyl-L-methionine (SAMe) and N-acetylcysteine (NAC) protective effects on hepatic damage when administered after acetaminophen overdose. Toxicology. 2008 Feb 3;244(1):25-34 pp.

50. Conn H.O., Bircher J. Hepatic encephalopathy: management with lactulose and related carbohydrates. 1988,1989: Medi-Ed Press. East Lansing, Michigan, 363 p.

51. Chugai Pharmaceutical Co. Improvements in and relating to L-ornithine-L-aspartate. U.K. Patent. 1,080,599, 1967.

52. "Hepa-Merz." Pharmacological description of the drug. Official site of "Merz." http://www.merz.ru/zabolevaniyapecheni/gepa-merc/

53. V.M. Frolov Glutargin: clinical efficacy and application prospects. Health of Ukraine (ukr). 2003; 78p.

54. Hensgens H.E.S.J. Regulation of urea cycle activity in rat liver ,.Ph.Diss. Amsterdam., Rodopi. 1980, 130 p.

55. McDermott W.V. Jr. The role of ammonia intoxication in hepatic coma. Bull N Y Acad Med. 1958 Jun;34(6):357-65 pp.

56. Schmid M., Peck-Radosavljevic M., König F., Mittermaier C., Gangl A., Ferenci P. A double-blind, randomized, placebo-controlled trial of intravenous L-ornithine-L-aspartate on postural control in patients with cirrhosis. Liver Int. 2010 Apr;30(4):574-82 pp.

57. Sikorska H., Cianciara J., Wiercińska-Drapało A. Physiological functions of L-ornithine and L-aspartate in the body and the efficacy of administration of L-ornithine-L-aspartate in conditions of relative deficiency. Pol Merkur Lekarski. 2010 Jun;28(168):490-5 pp.

58. Poo J.L., Góngora J., Sánchez-Avila F., Aguilar-Castillo S., García-Ramos G., Fernández-Zertuche M., Rodriguez-Fragoso L., Uribe M. Efficacy of oral L-ornithine-L-aspartate in cirrhotic patients with hyperammonemic hepatic encephalopathy. Results of a randomized, lactulose-controlled study. Ann Hepatol. 2006 Oct-Dec;5(4):281-8 pp.

59. Jiang Q., Jiang X.H., Zheng M.H., Chen Y.P. L-Ornithine-1-aspartate in the management of hepatic encephalopathy: a meta-analysis. J Gastroenterol Hepatol. 2009 Jan;24(1):9-14 pp.

60. Soárez P.C., Oliveira A.C., Padovan J., Parise E.R., Ferraz M.B. A critical analysis of studies assessing L-ornithine-L-aspartate (LOLA) in hepatic encephalopathy treatment. Arq Gastroenterol. 2009 Jul-Sep;46(3):241-7 pp.

61. 209. Acharya S.K., Bhatia V., Sreenivas V., Khanal S., Panda S.K. Efficacy of L-ornithine L-aspartate in acute liver failure: a double-blind, randomized, placebo-controlled study. Gastroenterology. 2009 Jun;136(7):2159-68 pp.

62. Soárez P.C., Oliveira A.C., Padovan J., Parise E.R., Ferraz M.B. A critical analysis of studies assessing L-ornithine-L-aspartate (LOLA) in hepatic encephalopathy treatment. Arq Gastroenterol. 2009 Jul-Sep;46(3):241-7 pp.

63. Yakovenko E.P., Labeznik L.B., A.V. Yakovenko, Lychkova A.E. A method for treating hepatic encephalopathy in liver cirrhosis. - M.: MIEMP, 17.06.2005;Pat. RU 2288711 C1, 10.12.2006.

64. Jalan R., Wright G., Davies N.A., Hodges S.J. L-Ornithine phenylacetate (OP): a novel treatment for hyperammonemia and hepatic encephalopathy. Med Hypotheses. 2007;69(5):1064-9 pp.

65. Malaguarnera M., Pistone G., Elvira R., Leotta C., Scarpello L., Liborio R. Effects of L-carnitine in patients with hepatic encephalopathy. World J Gastroenterol. 2005 Dec 7; 11(45):7197-202 pp.

66. Jones L.L., McDonald D.A., Borum P.R. Acylcarnitines: role in brain. Prog Lipid Res. 2010 Jan;49(1):61-75 pp.

67. Malaguarnera M., Gargante M.P., Cristaldi E., Vacante M., Risino C., Cammalleri L., Pennisi G., Rampello L. Acetyl-L-carnitine treatment in minimal hepatic encephalopathy. Dig Dis Sci. 2008 Nov;53(11):3018-25 pp.

68. Phosphogliv. Description of the preparation. http://www.piluli.ru/product/Fosfogliv

69. Kozhoka T.G., S.J. Jasinski Antihepatotoxic and hepatoprotective pharmaceutical composition and method for treating liver disease comprising administering to said composition. - M.: MIEMP, 2010, Pat. RU 2381800 C1, 20.02.2010.

70. O'Connor J.E., Costell M., Grisolia S. Prevention of ammonia toxicity by L-carnitine: metabolic changes in brain. Neurochem Res. 1984 Apr;9(4):563-70 pp.

71. Deshmukh D.R., Singh K.R., Meert K., Deshmukh G.D. Failure of L-carnitine to protect mice against hyperammonemia induced by ammonium acetate or urease injection. Pediatr Res. 1990 Sep;28(3):256-60 pp.

72. Kloiber O., Banjac B., Drewes L.R. Protection against acute hyperammonemia: the role of quaternary amines. Toxicology. 1988 Apr;49(1):83-90 pp.

73. Falchetto S., Kato G., Provini L. The action of carnitines on cortical neurons. Can J Physiol Pharmacol. 1971 Jan;49(1):1-7 pp.

74. Janiri L., Falcone M., Persico A., Tempesta E. Activity of L-carnitine and L-acetylcarnitine on cholinoceptive neocortical neurons of the rat in vivo. J Neural Transm Gen Sect. 1991;86(2):135-46 pp.

75. Dambrova M., Chlopicki S., Liepinsh E., Kirjanova O., Gorshkova O., Kozlovski V.I., Uhlen S., Liepina I., Petrovska R., Kalvinsh I. The methylester of gamma-butyrobetaine, but not gamma-butyrobetaine itself, induces muscarinic receptor-dependent vasodilatation. Naunyn Schmiedebergs Arch Pharmacol. 2004 May;369(5):533-9 pp.

76. Foster D.J., Heacock A.M., Keep R.F., Fisher S.K. Activation of muscarinic cholinergic receptors on human SH-SY5Y neuroblastoma cells enhances both the influx and efflux of K+ under conditions of hypo-osmolarity. J Pharmacol Exp Ther. 2008 May;325(2):457-65 pp.

77. Heacock A.M., Kerley D., Gurda G.T., VanTroostenberghe A.T., Fisher S.K. Potentiation of the osmosensitive release of taurine and D-aspartate from SH-SY5Y neuroblastoma cells after activation of M3 muscarinic cholinergic receptors. J Pharmacol Exp Ther. 2004 Dec;311(3):1097-104 pp.

78. Turovsky E.A., Turovsky M.N., Tolmacheva A.V., Zinchenko V.P., V.V .Dynnik The role of arginine in the regulation of alpha and beta adrenergic receptors in adipocytes. Collection of articles Conf. "Reception and intracellular signaling", Pushchino, ONTI Press, PSC RAS (Eds. Zinchenko V.P. et al), 2011, 95-99 pp.

79. O'Connor J.E., Guerri C., Jordá A., Grisolia S. On the mechanism of the protective effect of ethanol against ammonia intoxication in mice. Biochem Biophys Res Commun. 1982 Aug 31;107(4):1508-16 pp.

80. Nagy J. Alcohol related changes in regulation of NMDA receptor functions. Curr Neuropharmacol. 2008 Mar;6(1):39-54 pp.

81. Seligson D., McCormick G.J., Soborov V. Blood ketoglutarate and pyruvate in liver disease. J. Clin. Invest., 1952; 31: 661 p.

82. Walshe J.M. Observation on the symptomatology and pathogenesis of hepatic coma. J. Med. 1951; 20(4): 421-38 pp.

83. McDermott W.V. Jr., Adams R.D., Riddell A.G. Ammonia metabolism in man. Ann Surg. 1954 Oct;140(4):539-56 pp.

84. Bessman S.P. The rôle of ammonia in clinical syndromes. Ann Intern Med. 1956 May;44(5):1037-43 pp.

85. Dynnik V.V., Djafarov R.H., Djafarova I.M. Dynamics of aerobic energy metabolism. Coenzyme cycles, feedback and feedforward control and homeostasis. Dynamics of Biochemical Systems (Eds. Keleti T. et al),Budapest" Perg. Press, 1986; 185-202 pp.

86. Ataullakhanov F.I., Buravtsev V.N., Zhabotinskiĭ A.M., Norina S.B., Pichugin A.V. Interaction of the Embden-Meyerhof pathway and hexose monophosphate shunt in erythrocytes. Biokhimiia. 1981 Apr;46(4):723-31 pp.

87. Bergeron R., Coyle J.T., Tsai G., Greene R.W. NAAG reduces NMDA receptor current in CAI hippocampal pyramidal neurons of acute slices and dissociated neurons. NeurPsiPharm 2005;30:7-16 pp.

88. Neale S.A., Salt T.E. Modulation of GABAergic inhibition in the rat superior colliculus by a presynaptic group II metabotropic glutamate receptor. J Phisiol 2006; 577(2): 659-669 pp.

89. Glinski M.A. Asymmetrical dimethylarginine: metabolism, arginine paradox ,pathophysiology. Advances of Physiological Sciences (rus), 2007, 3: 21-39 pp.

90. Yeo T.W., Lampah D.A., Tjitra E., Gitawati R., Darcy C.J., Jones C., Kenangalem E., McNeil Y.R., Granger D.L., Lopansri B.K., Weinberg J.B., Price R.N., Duffull S.B., Celermajer D.S., Anstey N.M. Increased asymmetric dimethylarginine in severe falciparum malaria: association with impaired nitric oxide bioavailability and fatal outcome. PLoS Pathog. 2010 Apr 22;6(4):e1000868. Erratum in: PLoS Pathog. 2010;6(5). http://www.plospathogens.org/article/info%3Adoi%2F10.1371%2Fjournal.ppat.1000868

91. Richir M.C., Bouwman R.H., Teerlink T., Siroen M.P., de Vries T.P., van Leeuwen P.A. The prominent role of the liver in the elimination of asymmetric dimethylarginine (ADMA) and the consequences of impaired hepatic function. JPEN J Parenter Enteral Nutr. 2008 Nov-Dec;32(6):613-21 pp.

92. Gerova M., Török J. Hypotensive effect of arginine metabolite, is affected by NO synthase. Phisiol Res 2004; 53: 357-363 pp.

93. Tarasów E., Panasiuk A., Siergiejczyk L., Orzechowska-Bobkiewicz A., Lewszuk A., Walecki J., Prokopowicz D. MR and 1H MR spectroscopy of the brain in patients with liver cirrhosis and early stages of hepatic encephalopathy. Hepatogastroenterology. 2003 Nov-Dec;50(54):2149-53 pp.

94. Albrecht J., Zielińska M., Norenberg M.D. Glutamine as a mediator of ammonia neurotoxicity: A critical appraisal. Biochem Pharmacol. 2010 Nov 1;80(9):1303-8 pp.

95. Poveda M.J., Bernabeu A., Concepción L., Roa E., de Madaria E., Zapater P., Pérez-Mateo M., Jover R. Brain edema dynamics in patients with overt hepatic encephalopathy A magnetic resonance imaging study. Neuroimage. 2010 Aug 15;52(2):481-7 pp.

96. Kondrashova M.N .Signal properties of amber and ketoglutaric acids in the periodic interaction of the Krebs cycle with sympathetic- parasympathetic nervous system. Collection of articles conf. " Reception and intracellular signaling", Pushchino, ONTI Press, PSC RAS (Eds. Zinchenko V.P. et al), 2005 , 249-253 pp.

97. Succinic acid in medicine, food industry, agriculture : a collection of scientific articles. Ed. prof. Kondrasheva M.N., ONTI Press, PSC RAS , 1996 ,300p.

98. He W., Miao FJ, Lin DC, Schwandner RT, Wang Z, Gao J., Chen JL, Tian H., Ling L. Citric acid cycle intermediates as ligands for orphan G-protein-coupled receptors. Nature. 2004 May 13 , 429 (6988) :188-93 pp.

99. Lukyanova L.D. Signaling function of succinate, intracellular and intercellular reactions interaction during hypoxia. Collection of articles, conf. " Reception and intracellular signaling", ", Pushchino, ONTI Press, PSC RAS (Eds. Zinchenko V.P. et al), 2009 , 603-609 pp.

100. Ikeda H., Shiojima I., Oka T., Yoshida M., Maemura K., Walsh K., Igarashi T., Komuro I. Increased Akt-mTOR Signaling in Lung Epithelium Is Associated with Respiratory Distress Syndrome in Mice. Mol Cell Biol. 2011 Mar;31(5):1054-65 pp.

101. Dynnik V.V., Kosenko E.A., Akhmadieva A.H. Acute intoxication with ammonium as a model of hepatic coma. Effect of succinate , VINITI Press, Pushchino, 1988, 11p.

102. Schmidt L.E., Dalhoff K., Poulsen H.E. Acute versus chronic alcohol consumption in acetaminophen-induced hepatotoxicity. Hepatology. 2002 Apr;35(4):876-82 pp.

103. Bémeur C., Vaquero J., Desjardins P., Butterworth R.F. N-acetylcysteine attenuates cerebral complications of non-acetaminophen-induced acute liver failure in mice: antioxidant and antiinflammatory mechanisms. Metab Brain Dis. 2010 Jun;25(2):241-9 pp.

104. Saito C., Zwingmann C., Jaeschke H. Novel mechanisms of protection against acetaminophen hepatotoxicity in mice by glutathione and N-acetylcysteine. Hepatology. 2010 Jan;51(1):246-54 pp.

105. Wendel A., Jaeschke H. Drug-induced lipid peroxidation in mice--III. Glutathione content of liver, kidney and spleen after intravenous administration of free and liposomally entrapped glutathione. Biochem Pharmacol. 1982 Nov 15;31(22):3607-11 pp.

106. Littarru G.P., Tiano L. Bioenergetic and antioxidant properties of coenzyme Q10: recent developments. Mol Biotechnol. 2007 Sep;37(1):31-7 pp.

107. Rosenfeldt F.L., Haas S.J., Krum H., Hadj A., Ng K., Leong J.Y., Watts G.F. Coenzyme Q10 in the treatment of hypertension: a meta-analysis of the clinical trials. J Hum Hypertens. 2007 Apr;21(4):297-306 pp.

108. Chen X. Protective effects of quercetin on liver injury induced by ethanol. Pharmacogn Mag. 2010 Apr;6(22): 135-41 pp.

109. de Almeida L.M., Piñeiro C.C., Leite M.C., Brolese G., Tramontina F., Feoli A.M., Gottfried C., Gonçalves C.A. Resveratrol increases glutamate uptake, glutathione content, and S100B secretion in cortical astrocyte cultures.

Cell Mol Neurobiol. 2007 Aug;27(5):661-8 pp.

110. Hardy M. and all. S-Adenosyl-L-Methionine for treatment of depression, osteoarthritis, and liver disease. Agency for Healthcare Research and Quality (US); October 2002. ISBN-10: 1-58763-122-9.

111. Salinthone S., Yadav V., Bourdette D.N., Carr D.W. Lipoic acid: a novel therapeutic approach for multiple sclerosis and other chronic inflammatory diseases of the CNS. Endocr Metab Immune Disord Drug Targets. 2008 Jun;8(2):132-42 pp.

112. Shen W., Hao J., Feng Z., Tian C., Chen W., Packer L., Shi X., Zang W., Liu J. Lipoamide or lipoic acid stimulates mitochondrial biogenesis in 3T3-L1 adipocytes via the endothelial NO synthase-cGMP-protein kinase G signalling pathway. Br J Pharmacol. 2011 Mar;162(5):1213-1224 pp.

113. Li X., Liu Z., Luo C., Jia H., Sun L., Hou B., Shen W., Packer L., Cotman C.W., Liu J. Lipoamide protects retinal pigment epithelial cells from oxidative stress and mitochondrial dysfunction. Free Radic Biol Med. 2008 Apr 1;44(7):1465-74 pp.

114. Tuñón M.J., Alvarez M., Culebras J.M., González-Gallego J. An overview of animal models for investigating the pathogenesis and therapeutic strategies in acute hepatic failure. World J Gastroenterol. 2009 Jul 7;15(25):3086-98 pp.

115. Sawant S.P., Dnyanmote A.V., Shankar K., Limaye P.B., Latendresse J.R., Mehendale H.M. Potentiation of carbon tetrachloride hepatotoxicity and lethality in type 2 diabetic rats. J Pharmacol Exp Ther. 2004 Feb;308(2):694-704 pp.

116. Jayakumar A.R., Bethea J.R., Tong X.Y., Gomez J., Norenberg M.D. NF-κB in the mechanism of brain edema in acute liver failure: studies in transgenic mice. Neurobiol Dis. 2011 Feb;41(2):498-507 pp.

117. Junnila M., Ranco T., Sucura A., Lindberg L.-A. Reduction of carbon tetrachloride-indused hepatotoxic effects by oral administeration of betaine in male han-wistar rats: a morphometric histological study. Vet Pathol 2000; 37: 231-238 pp.

118. Boer L.A., Panatto J.P., Fagundes D.A., Bassani C., Jeremias I.C., Daufenbach J.F., Rezin G.T., Constantino L., Dal-Pizzol F., Streck E.L. Inhibition of mitochondrial respiratory chain in the brain of rats after hepatic failure induced by carbon tetrachloride is reversed by antioxidants. Brain Res Bull. 2009 Aug 28;80(1-2):75-8 pp.

119. Mikhail T.H., Awadallah R., Dessoukey E.A. Effect of AMP on acute carbon-tetrachloride hepatotoxicity. Z Ernahrungswiss 1977 ; 16:256-261 pp.

120. Stewart P.M., Walser M. Failure of the normal ureagenic response to amino acids in organic acid-loaded rats. Proposed mechanism for the hyperammonemia of propionic and methylmalonic acidemia. J Clin Invest. 1980 Sep;66(3):484-92 pp.

121. Coude F.X., Sweetman L., Nyhan W.L. Inhibition by propionyl-coenzyme A of N-acetylglutamate synthetase in rat liver mitochondria. A possible explanation for hyperammonemia in propionic and methylmalonic acidemia. J Clin Invest. 1979 Dec;64(6):1544-51 pp.

122. Jayakumar A.R., Bethea J.R., Tong X.Y., Gomez J., Norenberg M.D. NF-κB in the mechanism of brain edema in acute liver failure: studies in transgenic mice. Neurobiol Dis. 2011 Feb;41(2):498-507 pp.

123. Hernandes M.S., Troncone L.R. Glycine as a neurotransmitter in the forebrain: a short review. J Neural Transm. 2009 Dec;116(12):1551-60pp.

124. Schenker S., McCandless D.W., Brophy E., Lewis M.S. Studies on the intracerebral toxicity of ammonia. J Clin Invest. 1967 May;46(5):838-48pp.

125. Kosenko E.A., Venediktova N.I., Kudryavtsev A.A., Ataullakhanov F.I., Kaminsky Y.G., Felipo V., Montoliu C. Encapsulation of glutamine synthetase in mouse erythrocytes: a new procedure for ammonia detoxification. Biochem Cell Biol. 2008 Dec;86(6):469-76pp.

126. Kaminsky Y. G., Ataullakhanov F.I., Venediktova N.I., Kosenko E.A.,Marov N.V. A method for reducing the concentration of ammonia in the blood using using the ammocytes and encapsulated glutamine synthase , Moscow: MIEMP, 2009, Pat. RU 2007127114 A, 27.01.2009.

127. Braissant O. Ammonia toxicity to the brain: effects on creatine metabolism and transport and protective roles of creatine. Mol Genet Metab. 2010;100 Suppl 1:S53-8pp.

128. Izumi Y., Izumi M., Matsukawa M., Funatsu M., Zorumski C.F. Ammonia-mediated LTP inhibition: effects of NMDA receptor antagonists and L-carnitine. Neurobiol Dis. 2005 Nov;20(2):615-24pp.

129. Chu C.J., Wang S.S., Lee F.Y., Chang F.Y., Lin H.C., Hou M.C., Chan C.C., Wu S.L., Chen C.T., Huang H.C., Lee S.D. Detrimental effects of nitric oxide inhibition on hepatic encephalopathy in rats with thioacetamide-induced fulminant hepatic failure. Eur J Clin Invest. 2001 Feb;31(2):156-63pp.

130. Kilpatrick L.E., Polin R.A., Douglas S.D., Corkey B.E. Hepatic metabolic alterations in rats treated with low-dose endotoxin and aspirin: an animal model of Reye's syndrome. Metabolism. 1989 Jan;38(1):73-7pp.

**Claims**

1. Composition of protectors of acute and chronic hepatic encephalopathy ,containing per single dose: L- carnitine 4-6 g. , acetyl -L- carnitine 4-5g. , phosphocreatine 4-6 g. , N- acetyl cysteine 1-3g.,ethanol 20-50ml.

2. A pharmaceutical composition according to claim 1 prepared as a granules or as a powder, containing $NaHCO_3$ in equimolar to acidic compound doses and pharmaceutically acceptable diluents sweeteners and disaggregating agents and packed in sachet later to be dissolved in warm water and used as a beverage.

3. Composition of protectors of acute and chronic hepatic encephalopathy, containing per single dose: L- carnitine 1.5-3 g.; acetyl -L- carnitine 1-3 g.; succinate 0.75-1.5 g. ; L- glutamate 0.75-1.5 g. ; L- arginine 1,5-2,5 g, ;phosphocreatine 2-3g.;N- acetyl cysteine 0.25-1.5 g.;betaine 0.25-2g.;creatinine phosphate 0,25-1g.

4. A pharmaceutical composition according to claim 3 prepared as a granules or as a powder, containing $NaHCO_3$ in equimolar to acidic compound doses and pharmaceutically acceptable diluents sweeteners and disaggregating agents and packed in sachet later to be dissolved in warm water and used as a beverage.

5. Composition of protectors of acute and chronic hepatic encephalopathy ,containing per single dose; L- carnitine 2.5-3 g. ; acetyl -L- carnitine 0.5-1g.; Succinate 1-1.5 g. ;L- glutamate 1-1, 5 g.;L-arginine 1.5-2.5g.;phosphocreatine 1-3g.

6. A pharmaceutical composition according to claim 5 prepared as a granules or as a powder, containing $NaHCO_3$ in equimolar to acidic compound doses and pharmaceutically acceptable diluents sweeteners and disaggregating agents and packed in sachet later to be dissolved in warm water and used as a beverage.

7. Composition of protectors of acute and chronic hepatic encephalopathy ,containing per single dose : L- carnitine 1.5-3 g.; acetyl -L- carnitine 1-3 g.; Succinate 0.75-1.5 g.; , L- glutamate - 0.75-1.5 g.; , L- arginine 1,5-2,5 g.; phosphocreatine 2-3 g .; N- acetylcysteine 0.25-1.5 g.; betaine 0.25 2g.; , creatinine phosphate 0 ,25-1 g.; Coenzyme Q10 20-30mg and dihydroquercetin 15-20mg.

8. A pharmaceutical composition according to claim 7 prepared as a granules or as a powder, containing $NaHCO_3$ in equimolar to acidic compound doses and pharmaceutically acceptable diluents sweeteners and disaggregating agents and packed in sachet later to be dissolved in warm water and used as a beverage.

9. Composition of protectors of acute and chronic hepatic encephalopathy ,containing per single dose: L- carnitine 1.5-3 g.; of acetyl -L- carnitine 1-3 g.; Succinate 0.75-1.5 g.; , L- glutamate 0.75-1.5 g.; L- arginine 1,5-2,5 g.; phosphocreatine 2- 3g.; betaine 0.25- 2g.; creatinine phosphate 0.25-1 g.; Coenzyme Q10 =20 - 30mg.; dihydroquercetin = 15 - 20mg. and S- adenosylmethionine =0.1-0.5g.

10. A composition according to claim 9, **characterized in that** it additionally contains 0.03-0.1 g lipoamide.

11. A pharmaceutical composition according to claim 9 or 10 prepared as a granules or as a powder, containing $NaHCO_3$ in equimolar to acidic compound doses and pharmaceutically acceptable diluents sweeteners and disaggregating agents and packed in sachet later to be dissolved in warm water and used as a beverage.

12. A method of treating acute and chronic hepatic encephalopathy and liver diseases, selected from acute and chronic hepatitis, and steatohepatitis and cirrhosis of different etiology , comprising administering orally to patients for the treatment:

    - of acute stages of hepatic encephalopathy of the compositions ,according to claims 1 - 6 in an amount of from 1 to 3 doses per day in any combination, depending on the weight of the patient and individual tolerance of the components of the composition;
    of chronic stages of hepatic encephalopathy the compositions ,according to claims 7 - 10 in an amount of from 1 to 6 doses per day , in any combination depending on the weight of the patient and individual tolerance of the components of the claimed compositions.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 1080599 A
- RU 2288711 C1
- RU 2381800 C1
- RU 2007127114 A

### Non-patent literature cited in the description

- **BAJAJ S.** Review article: the modern management of hepatic encephalopathy. *Aliment Pharmacology Ther,* 2010, vol. 31 (5), 537-547
- **WOLF DC.** *Encephalopathy, hepatic. eMedicine Gastroenterology,* 18 August 2010, http://emedicine.medscape.com/article/186101-overview
- **SIBAE M.R. ; MCGUIRE B.M.** Current trends in the treatment of hepatic encephalopathy. *Ther Clin Risk Manag.,* June 2009, vol. 5 (3), 617-26
- **SEYAN A.S. ; HUGHES R.D. ; SHAWCROSS D.L.** Changing face of hepatic encephalopathy: role of inflammation and oxidative stress. *World J Gastroenterol.,* 21 July 2010, vol. 16 (27), 3347-3357
- **IVASHKIN V.T. ; NADINSKAYA M.Y. ; AO BUYEVEROV A.O.** Hepatic encephalopathy and its metabolic correction methods. *Bolez. org. indigestion (rus),* 2001, vol. 3, 25-7
- **STREKALOVA O.S. ; UCHAIKIN V.F. ; IPATOVA O.M. ; TORHOVSKAYA T.P. ; N.V .MEDVEDEV ; STOROZHAKOV G.I. ; ARCHAKOV, A.I.** Comatose states: etiopathogenesis, experimental study, treatment of hepatic coma. *Biomedical Chemistry,* 2009, vol. 55 (4), 380-396
- **BUTTERWORTH R.F.** Phatogenesis of hepatic encephalopathy: new insights from neuroimaging and molecular studies. *J Hepatol,* 2003, vol. 39, 278-285
- **BJERRING P.N. ; EEFSEN M. ; HANSEN B.A. ; LARSEN F.S.** The brain in acute liver failure. A tortuous path from hyperammonemia to cerebral edema. *Metab Brain Dis.,* March 2009, vol. 24 (1), 5-14
- **MARCAIDA G. ; MIÑANA M.D. ; BURGAL M. ; GRISOLIA S. ; FELIPO V.** Ammonia prevents activation of NMDA receptors by glutamate in rat cerebellar neuronal cultures. *Eur J Neurosci.,* 01 December 1995, vol. 7 (12), 2389-96
- **RODRIGO R ; CAULI O ; BOIX J ; ELMLILI N ; AGUSTI A ; FELIPO V.** Role of NMDA receptors in acute liver failure and ammonia toxicity: therapeutical implications. *Neurochem Int.,* July 2009, vol. 55 (1-3), 113-8

- **MONTOLIU C. ; LLANSOLA M. ; KOSENKO E. ; CORBALÁN R. ; FELIPO V.** Role of cyclic GMP in glutamate neurotoxicity in primary cultures of cerebellar neurons. *Neuropharmacology,* December 1999, vol. 38 (12), 1883-91
- **KOSENKO E.A. ; KAMINSKY Y.G .** Cellular mechanisms of toxicity of ammonia. *Moscow Publ LCI,* 2008, 288
- **BRUSILOW S.W. ; TRAYSTMAN R.** Hepatic encephalopathy. *N Engl J Med.,* 20 March 1986, vol. 314 (12), 786-7
- **ALBRECHT J. ; NORENBERG M.D.** Glutamine: a Trojan horse in ammonia neurotoxicity. *Hepatology,* October 2006, vol. 44 (4), 788-94
- **REINEHR R. ; GÖRG B. ; BECKER S. ; QVARTSKHAVA N. ; BIDMON H.J. ; SELBACH O. ; HAAS H.L. ; SCHLIESS F. ; HÄUSSINGER D.** Hypoosmotic swelling and ammonia increase oxidative stress by NADPH oxidase in cultured astrocytes and vital brain slices. *Glia,* May 2007, vol. 55 (7), 758-71
- **SINKE A.P. ; JAYAKUMAR A.R. ; PANICKAR K.S. ; MORIYAMA M. ; REDDY P.V. ; NORENBERG M.D.** NFkappaB in the mechanism of ammonia-induced astrocyte swelling in culture. *J Neurochem.,* September 2008, vol. 106 (6), 2302-11
- **JAYAKUMAR A.R. ; RAMA RAO K.V. ; TONG X.Y. ; NORENBERG M.D.** Calcium in the mechanism of ammonia-induced astrocyte swelling. *J Neurochem.,* May 2009, vol. 109 (1), 252-7
- **HILGIER W. ; WEGRZYNOWICZ M. ; RUSZKIEWICZ J. ; OJA S.S. ; SARANSAARI P. ; ALBRECHT J.** Direct exposure to ammonia and hyperammonemia increase the extracellular accumulation and degradation of astroglia-derived glutathione in the rat prefrontal cortex. *Toxicol Sci.,* September 2010, vol. 117 (1), 163-8
- **GEGG M.E. ; BELTRAN B. ; SALAS-PINO S. ; BOLANOS J.P. ; CLARK J.B. ; MONCADA.S. ; HEALES S.J.** Differential effect of nitric oxide on glutathione metabolism and mitochondrial function in astrocytes and neurones: implications for neuroprotection/neurodegeneration?. *J Neurochem.,* July 2003, vol. 86 (1), 228-37

- **SUÁREZ I. ; BODEGA G. ; FERNÁNDEZ B.** Glutamine synthetase in brain: effect of ammonia. *Neurochem Int.,* August 2002, vol. 41 (2-3), 123-42
- **KOSENKO E. ; LLANSOLA M. ; MONTOLIU C. ; MONFORT P. ; RODRIGO R. ; HERNANDEZ-VIA-DEL M. ; ERCEG S. ; SÁNCHEZ-PEREZ A.M. ; FELIPO V.** Glutamine synthetase activity and glutamine content in brain: modulation by NMDA receptors and nitric oxide. *Neurochemistry international,* 2003, vol. 43 (4-5), 493-9
- **SINGH S. ; TRIGUN S.K.** Activation of neuronal nitric oxide synthase in cerebellum of chronic hepatic encephalopathy rats is associated with up-regulation of NADPH-producing pathway. *Cerebellum,* September 2010, vol. 9 (3), 384-97
- **HÄUSSINGER D. ; GÖRG B.** Interaction of oxidative stress, astrocyte swelling and cerebral ammonia toxicity. *Curr Opin Clin Nutr Metab Care,* January 2010, vol. 13 (1), 87-92
- **MOSER H.** Electrophysiological evidence for ammonium as a substitute for potassium in activating the sodium pump in a crayfish sensory neuron. *Can J Physiol Pharmacol.,* February 1987, vol. 65 (2), 141-5
- **KEICHER E. ; MEECH R.** Endogenous Na(+)-K+ (or NH4+)-2Cl- cotransport in Rana oocytes; anomalous effect of external NH4+ on pHi. *J Physiol.,* 15 February 1994, vol. 475 (1), 45-57
- **KELLY T. ; KAFITZ K.W. ; RODERIGO C. ; ROSE C.R.** Ammonium-evoked alterations in intracellular sodium and pH reduce glial glutamate transport activity. *Glia,* 2009, vol. 57 (9), 921-34
- **KELLY T. ; ROSE C.R.** Ammonium influx pathways into astrocytes and neurones of hippocampal slices. *J Neurochem.,* December 2010, vol. 115 (5), 1123-36
- **DÍAZ-MUÑOZ M. ; TAPIA R.** Functional changes of brain mitochondria during experimental hepatic encephalopathy. *Biochem Pharmacol.,* 01 November 1989, vol. 38 (21), 3835-41
- **HAWKINS R.A. ; MILLER A.L. ; NIELSEN R.C. ; VEECH R.L.** The acute action of ammonia on rat brain metabolism in vivo. *Biochem J.,* August 1973, vol. 134 (4), 1001-8
- **KOSENKO E. ; KAMINSKY Y. ; GRAU E. ; MIÑANA M.D. ; MARCAIDA G. ; GRISOLIA S. ; FELIPO V.** Brain ATP depletion induced by acute ammonia intoxication in rats is mediated by activation of the NMDA receptor and Na+,K(+)-ATPase. *J Neurochem.,* 1994, vol. 63 (6), 2172-8
- Activation of $\alpha$2-adrenoceptors by L-arginine in cardiomyocytes SD and SHR rats. Collection of articles,Conf. **NENOV M.N. ; BEREZHNOV A.V. ; FEDOTOVA E.I. ; GRUSHIN K.S. ; O.J. PIMENOV ; SEMUSHINA S.G. ; PAKHOMOV LA. ; MURASHEV A.N . ; DYNNIKV.V. ; ZINCHENKO V.P.** Reception and intracellular signaling. ONTI Press, 2009, 143-148
- **NENOV M.N.** Regulation of L-type Ca2 + currents by L-arginine via $\alpha$2-adrenoceptor activation in isolated ventricular cardiomyocytes. *Ph. Diss.. biol. sciences,* 2009, 24
- **BASILE A.S. ; JONES E.A. ; SKOLNICK PH.** The pathogenesis and treatment of hepatic encephalopathy: evidence for the involvement of benzodiazepine receptor ligands. *Pharmacol Rev,* 1991, vol. 43 (1), 27-71
- **RADAD K. ; GILLE G. ; RAUSCH W.D.** Short review on dopamine agonists: insight into clinical and research studies relevant to Parkinson's disease. *Pharmacol Rep.,* November 2005, vol. 57 (6), 701-12
- **RATNAKUMARI L. ; QURESHI LA. ; MAYSINGER D. ; BUTTERWORTH R.F.** Developmental deficiency of the cholinergic system in congenitally hyperammonemic spf mice: effect of acetyl-L-carnitine. *J Pharmacol Exp Ther.,* July 1995, vol. 274 (1), 437-43
- **GARCÍA-AYLLÓN M.S. ; CAULI O. ; SILVEYRA M.X. ; RODRIGO R. ; CANDELA A. ; COMPAÑ A. ; JOVER R. ; PÉREZ-MATEO M. ; MARTINEZ S. ; FELIPO V.** Brain cholinergic impairment in liver failure. *Brain,* November 2008, vol. 131, 2946-56
- **TROST L.C. ; LEMASTERS J.J.** Reye's syndrome and related chemical toxicity. *Mitochondria in Pathogenesis,* 2002, vol. 5, 425-450
- **WARD M.R.** Reye's syndrome: an update. *Nurse Pract.,* December 1997, vol. 22 (12), 45-6, 49-50, 52-3
- Hepatic encephalopathy. Reyes syndrome. Toxic effects of fatty acids - ''four in one''. Collection of articles. **DYNNIK V.V. ; JAFAROV R..D ; GRISHIN E.V. ; V.A. KASSYMOV ; GRUSHIN K.S. ; KOKOZ Y.M. ; ZINCHENKO V.P. et al.** Conf. ''Reception and intracellular signaling. ONTI Press, 2005, 238-242
- **KNERR I. ; WEINHOLD N. ; VOCKLEY J. ; GIBSON K.M.** Advances and challenges in the treatment of branched-chain amino/keto acid metabolic defects. *J Inherit Metab Dis.,* 03 February 2011
- **DIONISI-VICI C. ; DEODATO F. ; RÖSCHINGER W. ; RHEAD W. ; WILCKEN B.** Classical' organic acidurias, propionic aciduria, methylmalonic aciduria and isovaleric aciduria: long-term outcome and effects of expanded newborn screening using tandem mass spectrometry. *J Inherit Metab Dis.,* April 2006, vol. 29 (2-3), 383-9

- **DHILLON H.S. ; DOSE J.M. ; SCHEFF S.W. ; PRASAD M.R.** Time course of changes in lactate and free fatty acids after experimental brain injury and relationship to morphologic damage. *Exp Neurol.,* July 1997, vol. 146 (1), 240-9
- **TAKEUCHI Y. ; MORII H. ; TAMURA M. ; HAYAISHI O. ; WATANABE Y.** A possible mechanism of mitochondrial dysfunction during cerebral ischemia: inhibition of mitochondrial respiration activity by arachidonic acid. *Arch Biochem Biophys.,* 15 August 1991, vol. 289 (1), 33-8
- Toxic effects of fatty acids. Role of phospholipases . Collection of articles. **BEREZHNOV A.V. ; FEDOTOVA E.I. ; NENOV M.N. ; V.P. ZINCHENKO ; V.V. DYNNIK et al.** Conf. '' Reception and intracellular signaling. ONTI Press, 2009, 15-19
- **BEREZHNOV A.V . ; FEDOTOVA E.I. ; NENOV M.N., V.P. ; ZINCHENKO , V.V.** Dynnik Calcium overload and cardiomyocytes death in the presence of activated fatty acids. *Contribution of phospholipases. Biological membranes (rus),* 2010, vol. 27 (1), 67-76
- **BEREZHNOV AV.** Investigation of the mechanisms of acute toxic effects of acylcarnithines : Ph. Diss. biol. Sciences. *IBK RAS,* 2009
- **BHATIA V. ; SINGH R. ; ACHARYA S.K.** Predictive value of arterial ammonia for complications and outcome in acute liver failure. *Gut,* June 2006, vol. 55 (1), 98-104
- **KAMINSKY Y. ; KOSENKO E.** Brain purine metabolism and xanthine dehydrogenase/oxidase conversion in hyperammonemia are under control of NMDA receptors and nitric oxide. *Brain Res.,* 19 October 2009, vol. 1294, 193-201
- **TERNEUS M.V. ; BROWN J.M. ; CARPENTER A.B. ; VALENTOVIC M.A.** Comparison of S-adenosyl-L-methionine (SAMe) and N-acetylcysteine (NAC) protective effects on hepatic damage when administered after acetaminophen overdose. *Toxicology,* 03 February 2008, vol. 244 (1), 25-34
- **CONN H.O. ; BIRCHER J.** Hepatic encephalopathy: management with lactulose and related carbohydrates. Medi-Ed Press, 1988, 363
- **V.M. FROLOV.** *Glutargin: clinical efficacy and application prospects,* 2003, 78
- **HENSGENS H.E.S.J.** Regulation of urea cycle activity in rat liver. *Ph.Diss.,* 1980, 130
- **MCDERMOTT W.V. JR.** The role of ammonia intoxication in hepatic coma. *Bull N Y Acad Med.,* June 1958, vol. 34 (6), 357-65
- **SCHMID M. ; PECK-RADOSAVLJEVIC M. ; KÖNIG F. ; MITTERMAIER C. ; GANGL A. ; FERENCI P.** A double-blind, randomized, placebo-controlled trial of intravenous L-ornithine-L-aspartate on postural control in patients with cirrhosis. *Liver Int.,* April 2010, vol. 30 (4), 574-82
- **POO J.L. ; GÓNGORA J. ; SÁNCHEZ-AVILA F. ; AGUILAR-CASTILLO S. ; GARCÍA-RAMOS G. ; FERNÁNDEZ-ZERTUCHE M. ; RODRIGUEZ-FRAGOSO L. ; URIBE M.** Efficacy of oral L-ornithine-L-aspartate in cirrhotic patients with hyperammonemic hepatic encephalopathy. Results of a randomized, lactulose-controlled study. *Ann Hepatol.,* October 2006, vol. 5 (4), 281-8
- **JIANG Q. ; JIANG X.H. ; ZHENG M.H. ; CHEN Y.P.** L-Ornithine-1-aspartate in the management of hepatic encephalopathy: a meta-analysis. *J Gastroenterol Hepatol.,* January 2009, vol. 24 (1), 9-14
- **SOÁREZ P.C. ; OLIVEIRA A.C. ; PADOVAN J. ; PARISE E.R. ; FERRAZ M.B.** A critical analysis of studies assessing L-ornithine-L-aspartate (LOLA) in hepatic encephalopathy treatment. *Arq Gastroenterol.,* July 2009, vol. 46 (3), 241-7
- **ACHARYA S.K. ; BHATIA V. ; SREENIVAS V. ; KHANAL S. ; PANDA S.K.** Efficacy of L-ornithine L-aspartate in acute liver failure: a double-blind, randomized, placebo-controlled study. *Gastroenterology,* June 2009, vol. 136 (7), 2159-68
- **YAKOVENKO E.P. ; LABEZNIK L.B. ; A.V. YAKOVENKO ; LYCHKOVA A.E.** A method for treating hepatic encephalopathy in liver cirrhosis. - M. *MIEMP,* 17 June 2005
- **JALAN R. ; WRIGHT G. ; DAVIES N.A. ; HODGES S.J.** L-Ornithine phenylacetate (OP): a novel treatment for hyperammonemia and hepatic encephalopathy. *Med Hypotheses,* 2007, vol. 69 (5), 1064-9
- **MALAGUARNERA M. ; PISTONE G. ; ELVIRA R. ; LEOTTA C. ; SCARPELLO L. ; LIBORIO R.** Effects of L-carnitine in patients with hepatic encephalopathy. *World J Gastroenterol.,* 07 December 2005, vol. 11 (45), 7197-202
- **JONES L.L. ; MCDONALD D.A. ; BORUM P.R.** Acylcarnitines: role in brain. *Prog Lipid Res.,* January 2010, vol. 49 (1), 61-75
- **MALAGUARNERA M. ; GARGANTE M.P. ; CRISTALDI E. ; VACANTE M. ; RISINO C. ; CAMMALLERI L. ; PENNISI G. ; RAMPELLO L.** Acetyl-L-carnitine treatment in minimal hepatic encephalopathy. *Dig Dis Sci.,* November 2008, vol. 53 (11), 3018-25
- **KOZHOKA T.G. ; S.J. JASINSKI.** Antihepatotoxic and hepatoprotective pharmaceutical composition and method for treating liver disease comprising administering to said composition. - M. *MIEMP,* 2010
- **O'CONNOR J.E. ; COSTELL M. ; GRISOLIA S.** Prevention of ammonia toxicity by L-carnitine: metabolic changes in brain. *Neurochem Res.,* April 1994, vol. 9 (4), 563-70

- **DESHMUKH D.R. ; SINGH K.R. ; MEERT K. ; DESHMUKH G.D.** Failure of L-carnitine to protect mice against hyperammonemia induced by ammonium acetate or urease injection. *Pediatr Res.,* September 1990, vol. 28 (3), 256-60
- **KLOIBER O. ; BANJAC B. ; DREWES L.R.** Protection against acute hyperammonemia: the role of quaternary amines. *Toxicology,* April 1988, vol. 49 (1), 83-90
- **FALCHETTO S. ; KATO G. ; PROVINI L.** The action of carnitines on cortical neurons. *Can J Physiol Pharmacol.,* January 1971, vol. 49 (1), 1-7
- **JANIRI L. ; FALCONE M. ; PERSICO A. ; TEMPESTA E.** Activity of L-carnitine and L-acetylcarnitine on cholinoceptive neocortical neurons of the rat in vivo. *J Neural Transm Gen Sect.,* 1991, vol. 86 (2), 135-46
- **DAMBROVA M. ; CHLOPICKI S. ; LIEPINSH E. ; KIRJANOVA O. ; GORSHKOVA O. ; KOZLOVSKI V.I. ; UHLEN S. ; LIEPINA I. ; PETROVSKA R. ; KALVINSH I.** The methylester of gamma-butyrobetaine, but not gamma-butyrobetaine itself, induces muscarinic receptor-dependent vasodilatation. *Naunyn Schmiedebergs Arch Pharmacol.,* May 2004, vol. 369 (5), 533-9
- **FOSTER D.J. ; HEACOCK A.M. ; KEEP R.F. ; FISHER S.K.** Activation of muscarinic cholinergic receptors on human SH-SY5Y neuroblastoma cells enhances both the influx and efflux of K+ under conditions of hypo-osmolarity. *J Pharmacol Exp Ther.,* May 2008, vol. 325 (2), 457-65
- **HEACOCK A.M. ; KERLEY D. ; GURDA G.T. ; VANTROOSTENBERGHE A.T. ; FISHER S.K.** Potentiation of the osmosensitive release of taurine and D-aspartate from SH-SY5Y neuroblastoma cells after activation of M3 muscarinic cholinergic receptors. *J Pharmacol Exp Ther.,* December 2004, vol. 311 (3), 1097-104
- The role of arginine in the regulation of alpha and beta adrenergic receptors in adipocytes. Collection of article. **TUROVSKY E.A. ; TUROVSKY M.N. ; TOLMACHEVA A.V. ; ZINCHENKO V.P. ; V.V .DYNNIK et al.** Conf. ''Reception and intracellular signaling. ONTI Press, 2011, 95-99
- **O'CONNOR J.E. ; GUERRI C. ; JORDÁ A. ; GRISOLIA S.** On the mechanism of the protective effect of ethanol against ammonia intoxication in mice. *Biochem Biophys Res Commun,* 31 August 1982, vol. 107 (4), 1508-16
- **NAGY J.** Alcohol related changes in regulation of NMDA receptor functions. *Curr Neuropharmacol.,* March 2008, vol. 6 (1), 39-54
- **SELIGSON D. ; MCCORMICK G.J. ; SOBOROV V.** Blood ketoglutarate and pyruvate in liver disease. *J. Clin. Invest.,* 1952, vol. 31, 661
- **WALSHE J.M.** Observation on the symptomatology and pathogenesis of hepatic coma. *J. Med.,* 1951, vol. 20 (4), 421-38
- **MCDERMOTT W.V. JR. ; ADAMS R.D. ; RIDDELL A.G.** Ammonia metabolism in man. *Ann Surg.,* October 1954, vol. 140 (4), 539-56
- **BESSMAN S.P.** The rôle of ammonia in clinical syndromes. *Ann Intern Med.,* 1956, vol. 44 (5), 1037-43
- Dynamics of aerobic energy metabolism. Coenzyme cycles, feedback and feedforward control and homeostasis. **DYNNIK V.V. ; DJAFAROV R.H. ; DJAFAROVA I.M. et al.** Dynamics of Biochemical Systems. Perg. Press, 1986, 185-202
- **ATAULLAKHANOV F.I. ; BURAVTSEV V.N. ; ZHABOTINSKIĬ A.M. ; NORINA S.B. ; PICHUGIN A.V.** Interaction of the Embden-Meyerhof pathway and hexose monophosphate shunt in erythrocytes. *Biokhimiia,* April 1981, vol. 46 (4), 723-31
- **BERGERON R. ; COYLE J.T. ; TSAI G. ; GREENE R.W.** NAAG reduces NMDA receptor current in CAI hippocampal pyramidal neurons of acute slices and dissociated neurons. *NeurPsiPharm,* 2005, vol. 30, 7-16
- **NEALE S.A. ; SALT T.E.** Modulation of GABAergic inhibition in the rat superior colliculus by a presynaptic group II metabotropic glutamate receptor. *J Phisiol,* 2006, vol. 577 (2), 659-669
- **GLINSKI M.A.** Asymmetrical dimethylarginine: metabolism, arginine paradox ,pathophysiology. *Advances of Physiological Sciences,* 2007, vol. 3, 21-39
- **YEO T.W. ; LAMPAH D.A. ; TJITRA E. ; GITAWATI R. ; DARCY C.J. ; JONES C. ; KENANGALEM E. ; MCNEIL Y.R. ; GRANGER D.L. ; LOPANSRI B.K.** Increased asymmetric dimethylarginine in severe falciparum malaria: association with impaired nitric oxide bioavailability and fatal outcome. *PLoS Pathog.,* 22 April 2010, vol. 6 (4), e1000868
- **ERRATUM.** *PLoS Pathog.,* 2010, vol. 6 (5, http://www.plospathogens.org/article/info%3Adoi%2F10.1371%2Fjournal.ppat.1000868
- **RICHIR M.C. ; BOUWMAN R.H. ; TEERLINK T. ; SIROEN M.P. ; DE VRIES T.P. ; VAN LEEUWEN P.A.** The prominent role of the liver in the elimination of asymmetric dimethylarginine (ADMA) and the consequences of impaired hepatic function. *JPEN J Parenter Enteral Nutr.,* November 2008, vol. 32 (6), 613-21
- **GEROVA M. ; TÖRÖK J.** Hypotensive effect of arginine metabolite, is affected by NO synthase. *Phisiol Res,* 2004, vol. 53, 357-363
- **TARASÓW E. ; PANASIUK A. ; SIERGIEJCZYK L. ; ORZECHOWSKA-BOBKIEWICZ A. ; LEWSZUK A. ; WALECKI J. ; PROKOPOWICZ D.** MR and 1H MR spectroscopy of the brain in patients with liver cirrhosis and early stages of hepatic encephalopathy. *Hepatogastroenterology,* November 2003, vol. 50 (54), 2149-53

- **ALBRECHT J. ; ZIELIŃSKA M. ; NORENBERG M.D.** Glutamine as a mediator of ammonia neurotoxicity: A critical appraisal. *Biochem Pharmacol.,* 01 November 2010, vol. 80 (9), 1303-8
- **POVEDA M.J. ; BERNABEU A. ; CONCEPCIÓN L. ; ROA E. ; DE MADARIA E. ; ZAPATER P. ; PÉREZ-MATEO M. ; JOVER R.** Brain edema dynamics in patients with overt hepatic encephalopathy A magnetic resonance imaging study. *Neuroimage,* 15 August 2010, vol. 52 (2), 481-7
- Signal properties of amber and ketoglutaric acids in the periodic interaction of the Krebs cycle with sympathetic- parasympathetic nervous system. **KONDRASHOVA M.N et al.** Collection of articles conf. '' Reception and intracellular signaling. ONTI Press, 2005, 249-253
- Succinic acid in medicine, food industry, agriculture : a collection of scientific articles. ONTI Press, 1996, 300
- **HE W. ; MIAO FJ ; LIN DC ; SCHWANDNER RT ; WANG Z. ; GAO J. ; CHEN JL ; TIAN H. ; LING L.** Citric acid cycle intermediates as ligands for orphan G-protein-coupled receptors. *Nature,* 13 May 2004, vol. 429 (6988), 188-93
- Signaling function of succinate, intracellular and intercellular reactions interaction during hypoxia. **LUKYANOVA L.D. et al.** Collection of articles, conf. '' Reception and intracellular signaling. ONTI Press, 2009, 603-609
- **IKEDA H. ; SHIOJIMA I. ; OKA T. ; YOSHIDA M. ; MAEMURA K. ; WALSH K. ; IGARASHI T. ; KOMURO I.** Increased Akt-mTOR Signaling in Lung Epithelium Is Associated with Respiratory Distress Syndrome in Mice. *Mol Cell Biol.,* March 2011, vol. 31 (5), 1054-65
- **DYNNIK V.V. ; KOSENKO E.A. ; AKHMADIEVA A.H.** Acute intoxication with ammonium as a model of hepatic coma. *Effect of succinate,* 1988, 11
- **SCHMIDT L.E. ; DALHOFF K. ; POULSEN H.E.** Acute versus chronic alcohol consumption in acetaminophen-induced hepatotoxicity. *Hepatology,* April 2002, vol. 35 (4), 876-82
- **BÉMEUR C. ; VAQUERO J. ; DESJARDINS P. ; BUTTERWORTH R.F.** N-acetylcysteine attenuates cerebral complications of non-acetaminophen-induced acute liver failure in mice: antioxidant and antiinflammatory mechanisms. *Metab Brain Dis.,* June 2010, vol. 25 (2), 241-9
- **SAITO C. ; ZWINGMANN C. ; JAESCHKE H.** Novel mechanisms of protection against acetaminophen hepatotoxicity in mice by glutathione and N-acetylcysteine. *Hepatology,* January 2010, vol. 51 (1), 246-54
- **WENDEL A. ; JAESCHKE H.** Drug-induced lipid peroxidation in mice--III. Glutathione content of liver, kidney and spleen after intravenous administration of free and liposomally entrapped glutathione. *Biochem Pharmacol.,* 15 November 1982, vol. 31 (22), 3607-11
- **LITTARRU G.P. ; TIANO L.** Bioenergetic and antioxidant properties of coenzyme Q10: recent developments. *Mol Biotechnol.,* September 2007, vol. 37 (1), 31-7
- **ROSENFELDT F.L. ; HAAS S.J. ; KRUM H. ; HADJ A. ; NG K. ; LEONG J.Y. ; WATTS G.F.** Coenzyme Q10 in the treatment of hypertension: a meta-analysis of the clinical trials. *J Hum Hypertens,* April 2007, vol. 21 (4), 297-306
- **CHEN X.** Protective effects of quercetin on liver injury induced by ethanol. *Pharmacogn Mag.,* April 2010, vol. 6 (22), 135-41
- **DE ALMEIDA L.M. ; PIÑEIRO C.C. ; LEITE M.C. ; BROLESE G. ; TRAMONTINA F. ; FEOLI A.M. ; GOTTFRIED C. ; GONÇALVES C.A.** Resveratrol increases glutamate uptake, glutathione content, and S100B secretion in cortical astrocyte cultures. *Cell Mol Neurobiol.,* August 2007, vol. 27 (5), 661-8
- **HARDY M.** S-Adenosyl-L-Methionine for treatment of depression, osteoarthritis, and liver disease. *Agency for Healthcare Research and Quality (US),* October 2002, ISBN 10: 1-58763-122-9
- **SALINTHONE S. ; YADAV V. ; BOURDETTE D.N. ; CARR D.W.** Lipoic acid: a novel therapeutic approach for multiple sclerosis and other chronic inflammatory diseases of the CNS. *Endocr Metab Immune Disord Drug Targets,* June 2008, vol. 8 (2), 132-42
- **SHEN W. ; HAO J. ; FENG Z. ; TIAN C. ; CHEN W. ; PACKER L. ; SHI X. ; ZANG W. ; LIU J.** Lipoamide or lipoic acid stimulates mitochondrial biogenesis in 3T3-L1 adipocytes via the endothelial NO synthase-cGMP-protein kinase G signalling pathway. *Br J Pharmacol.,* March 2011, vol. 162 (5), 1213-1224
- **LI X. ; LIU Z. ; LUO C. ; JIA H. ; SUN L. ; HOU B. ; SHEN W. ; PACKER L. ; COTMAN C.W. ; LIU J.** Lipoamide protects retinal pigment epithelial cells from oxidative stress and mitochondrial dysfunction. *Free Radic Biol Med.,* 01 April 2008, vol. 44 (7), 1465-74
- **TUÑÓN M.J. ; ALVAREZ M. ; CULEBRAS J.M. ; GONZÁLEZ-GALLEGO J.** An overview of animal models for investigating the pathogenesis and therapeutic strategies in acute hepatic failure. *World J Gastroenterol.,* 07 July 2009, vol. 15 (25), 3086-98

- **SAWANT S.P. ; DNYANMOTE A.V. ; SHANKAR K. ; LIMAYE P.B. ; LATENDRESSE J.R. ; MEHENDALE H.M.** Potentiation of carbon tetrachloride hepatotoxicity and lethality in type 2 diabetic rats. *J Pharmacol Exp Ther.,* February 2004, vol. 308 (2), 694-704
- **JAYAKUMAR A.R. ; BETHEA J.R. ; TONG X.Y. ; GOMEZ J. ; NORENBERG M.D.** NF-κB in the mechanism of brain edema in acute liver failure: studies in transgenic mice. *Neurobiol Dis.,* February 2011, vol. 41 (2), 498-507
- **JUNNILA M. ; RANCO T. ; SUCURA A. ; LINDBERG L.-A.** Reduction of carbon tetrachloride-indused hepatotoxic effects by oral administeration of betaine in male han-wistar rats: a morphometric histological study. *Vet Pathol,* 2000, vol. 37, 231-238
- **BOER L.A. ; PANATTO J.P. ; FAGUNDES D.A. ; BASSANI C. ; JEREMIAS I.C. ; DAUFENBACH J.F. ; REZIN G.T. ; CONSTANTINO L. ; DAL-PIZZOL F. ; STRECK E.L.** Inhibition of mitochondrial respiratory chain in the brain of rats after hepatic failure induced by carbon tetrachloride is reversed by antioxidants. *Brain Res Bull.,* 28 August 2009, vol. 80 (1-2), 75-8
- **MIKHAIL T.H. ; AWADALLAH R. ; DESSOUKEY E.A.** Effect of AMP on acute carbon-tetrachloride hepatotoxicity. *Z Ernahrungswiss,* 1997, vol. 16, 256-261
- **STEWART P.M. ; WALSER M.** Failure of the normal ureagenic response to amino acids in organic acid-loaded rats. Proposed mechanism for the hyperammonemia of propionic and methylmalonic acidemia. *J Clin Invest.,* September 1980, vol. 66 (3), 484-92
- **COUDE F.X. ; SWEETMAN L. ; NYHAN W.L.** Inhibition by propionyl-coenzyme A of N-acetylglutamate synthetase in rat liver mitochondria. A possible explanation for hyperammonemia in propionic and methylmalonic acidemia. *J Clin Invest.,* December 1979, vol. 64 (6), 1544-51
- **HERNANDES M.S. ; TRONCONE L.R.** Glycine as a neurotransmitter in the forebrain: a short revie. *J Neural Transm.,* December 2009, vol. 116 (12), 1551-60
- **SCHENKER S. ; MCCANDLESS D.W. ; BROPHY E. ; LEWIS M.S.** Studies on the intracerebral toxicity of ammonia. *J Clin Invest.,* May 1967, vol. 46 (5), 838-48
- **KOSENKO E.A. ; VENEDIKTOVA N.I. ; KUDRYAVTSEV A.A. ; ATAULLAKHANOV F.I. ; KAMINSKY Y.G. ; FELIPO V. ; MONTOLIU C.** Encapsulation of glutamine synthetase in mouse erythrocytes: a new procedure for ammonia detoxification. *Biochem Cell Biol.,* December 2008, vol. 86 (6), 469-76
- **KAMINSKY Y. G. ; ATAULLAKHANOV F.I. ; VENEDIKTOVA N.I. ; KOSENKO E.A. ; MAROV N.V.** A method for reducing the concentration of ammonia in the blood using using the ammocytes and encapsulated glutamine synthase. *MIEMP,* 2009
- **BRAISSANT O.** Ammonia toxicity to the brain: effects on creatine metabolism and transport and protective roles of creatine. *Mol Genet Metab.,* 2010, vol. 100 (1), 53-8
- **IZUMI Y. ; IZUMI M. ; MATSUKAWA M. ; FUNATSU M. ; ZORUMSKI C.F.** Ammonia-mediated LTP inhibition: effects of NMDA receptor antagonists and L-carnitine. *Neurobiol Dis.,* November 2005, vol. 20 (2), 615-24
- **CHU C.J. ; WANG S.S. ; LEE F.Y. ; CHANG F.Y. ; LIN H.C. ; HOU M.C. ; CHAN C.C. ; WU S.L. ; CHEN C.T. ; HUANG H.C.** Detrimental effects of nitric oxide inhibition on hepatic encephalopathy in rats with thioacetamide-induced fulminant hepatic failure. *Eur J Clin Invest.,* February 2001, vol. 31 (2), 156-63
- **KILPATRICK L.E. ; POLIN R.A. ; DOUGLAS S.D. ; CORKEY B.E.** Hepatic metabolic alterations in rats treated with low-dose endotoxin and aspirin: an animal model of Reye's syndrome. *Metabolism.,* January 1989, vol. 38 (1), 73-7